# EUROPEAN PATENT APPLICATION

(11) **EP 4 470 608 A1**
(43) Date of publication of application: **04.12.2024**
(21) Application number: 23747083.6
(22) Date of filing: 27.01.2023
(51) Int. Cl.: A61P 1/00, A61P 1/18, A61P 9/00, A61P 11/00, A61P 13/12, A61P 27/02, A61P 17/00, C07F 9/09, A61P 7/00, A61K 31/685, A61P 25/28

(54) **LYSOPHOSPHATIDYLSERINE ANALOGUE, AND LYSOPHOSPHATIDYLSERINE ANALOGUE-CONTAINING PHAMACEUTICAL COMPOSITION FOR TREATING OR PREVENTING FIBROSIS**

(30) Priority: 27.01.2022 US 202263303564 P
(71) Applicant: The University of Tokyo, Bunkyo-ku, Tokyo 113-8654 (JP)
(72) Inventor: OHWADA, Tomohiko, Tokyo 113-8654 (JP); OTANI, Yuko, Tokyo 113-8654 (JP); CHEN, Luying, Tokyo 113-8654 (JP); MIYAJIMA, Atsushi, Tokyo 113-8654 (JP); KIDO, Taketomo, Tokyo 113-8654 (JP)
(74) Representative: D Young & Co LLP
(86) International application number: PCT/JP2023/002625
(87) International publication number: WO 2023/145873

(57) **Abstract**

The present invention relates to a compound represented by the following Formula (I) or a pharmaceutically acceptable salt thereof: 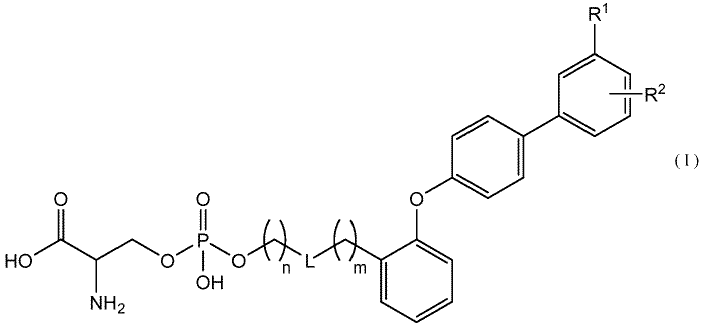 wherein in Formula (I), definitions of R¹, R², L, m, and n are each shown in the specification.

## Description

### Technical Field

The present invention relates to a lysophosphatidylserine analogue and a pharmaceutical composition for treating or preventing fibrosis including a lysophosphatidylserine analogue.

### Background Art

Fibrosis is a disease state in which extracellular matrices such as collagen are excessively deposited and cause dysfunction of organs and the like. Fibrosis is commonly observed in various diseases such as cancer, circulatory diseases caused by diabetes, hypertension, dyslipidemia and the like, chronic kidney disease, chronic obstructive pulmonary disease, and alcoholic and non-alcoholic fatty liver diseases (including NASH and NAFLD). Furthermore, fibrosis in organs such as liver, pancreas, and lungs also becomes the origin of cancer.

Fibrosing that causes fibrosis is one of the wound healing processes and occurs in various organs such as the heart, eye, brain, digestive organs, skin, lung, kidney, hematopoietic organs, retroperitoneum, mediastinum, and joints. The treatment and prevention of fibrosis is an important issue, since excessive fibrosing causes dysfunction of organs and progresses pathogenesis.

In addition, Non Patent Literature 1 discloses that lysophosphatidylserine (LysoPS), which is one of lysophospholipids, has an action of promoting migration of fibroblasts.

### Citation List

### Non Patent Literature

Non Patent Literature 1: Park, K. S. et al., Mol. Pharmacol. 2006, 69 (3), 1066-1073.

### Summary of Invention

### Technical Problem

However, an effective treatment method for fibrosis has not been established, and development of a medicine capable of treating or preventing fibrosis is desired.

An object of the present invention is to provide a novel compound that can be used for treatment or prevention of fibrosis.

### Solution to Problem

The present inventors conducted intensive studies, and as a result, the inventors found a novel compound as a compound effective for the treatment or prevention of fibrosis, thus completing the present invention.

That is, the present invention is as follows.
[1] A compound represented by the following Formula (I) or a pharmaceutically acceptable salt thereof: 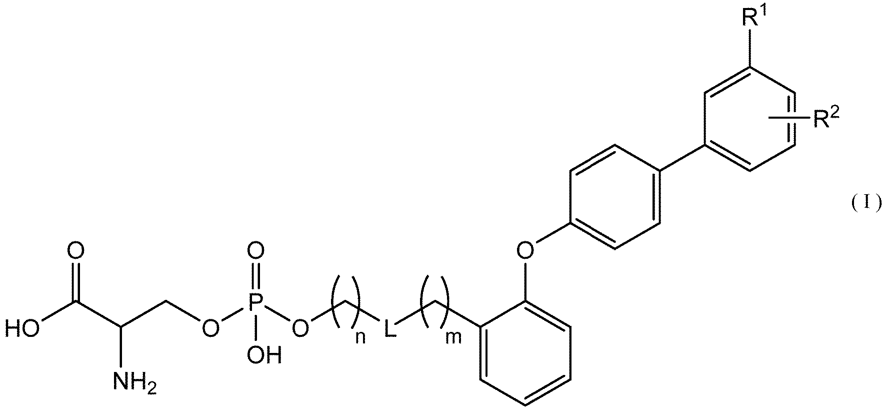 wherein in Formula (I),
   L represents -O-, -NR-, -S-, -(C=O)O-, -O(C=O)-, - (C=O)NR-, -NR(C=O)-, -(C=S)NR-, -NR(C=S)-, -(C=O)S-, - S(C=O)-, -(C=S)O-, -O(C=S)-, (C=S)S-, or -S(C=S)-;
   R represents a hydrogen atom or an aliphatic hydrocarbon group having 1 to 10 carbon atoms which may have a substituent;
   R¹ and R² each independently represent an aliphatic hydrocarbon group having 1 to 10 carbon atoms which may have a substituent, an aromatic hydrocarbon group which may have a substituent, or -OR³;
   R³ represents an aliphatic hydrocarbon group having 1 to 10 carbon atoms which may have a substituent, or an aromatic hydrocarbon group which may have a substituent; and
   m and n are each independently an integer of 1 to 6.
[2] The compound according to [1], or a pharmaceutically acceptable salt thereof, wherein R² is located at the meta-position with respect to the bonding position of R¹.
[3] The compound according to [1] or [2], or a pharmaceutically acceptable salt thereof, wherein R¹ and R² are identical.
[4] The compound according to any one of [1] to [3], or a pharmaceutically acceptable salt thereof, wherein R¹ and R² are each independently ethyl, isopropyl, t-butyl, phenyl, methylphenyl, benzyl, trifluoromethyl, or methoxy.
[5] A compound represented by the following Formula (II) or the following Formula (III), or a pharmaceutically acceptable salt thereof. 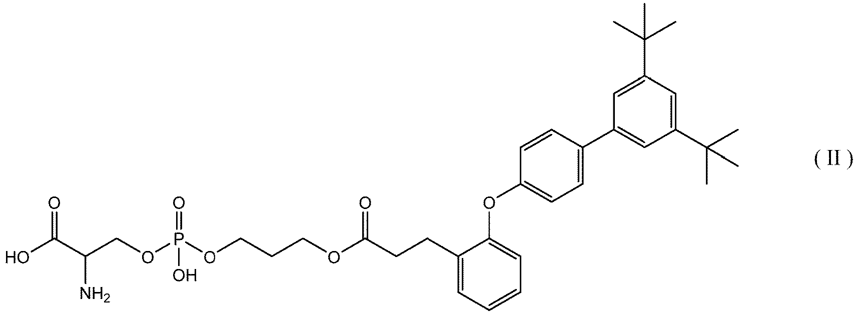 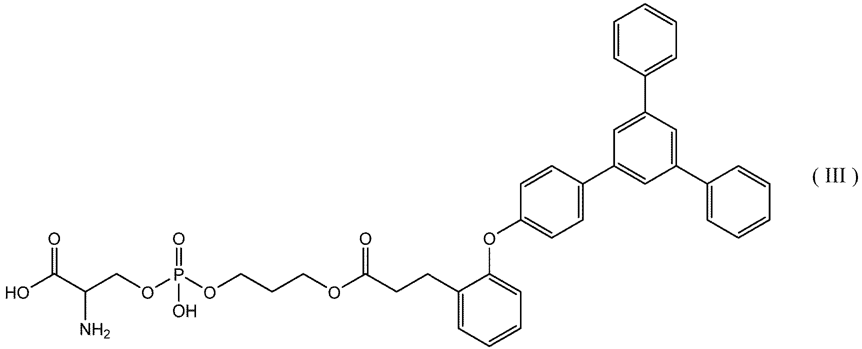
[6] A pharmaceutical composition for treating or preventing fibrosis, containing the compound according to any one of [1] to [5] or a pharmaceutically acceptable salt thereof.
[7] A pharmaceutical composition for treating or preventing fibrosis, containing a compound represented by the following Formula (II) or the following Formula (III), or a pharmaceutically acceptable salt thereof. 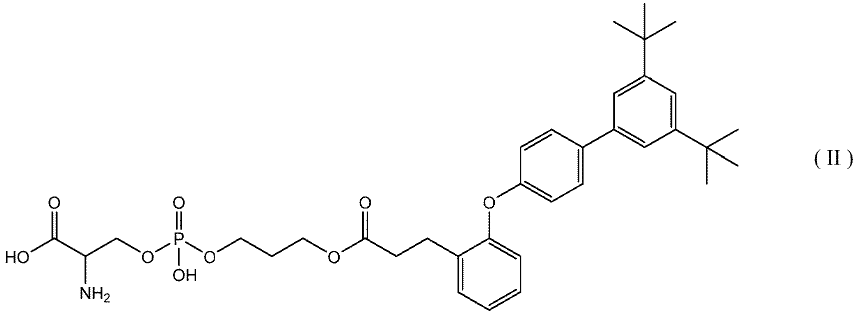 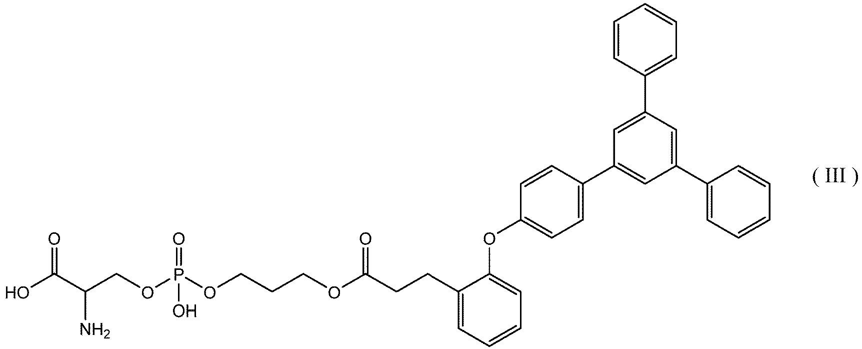
[8] The pharmaceutical composition according to [6] or [7], wherein the fibrosis is fibrosis in a heart, an eye, a brain, a digestive organ, a skin, a lung, a kidney, a hematopoietic organ, a retroperitoneum, a mediastinum, or a joint.
[9] The pharmaceutical composition according to any one of [6] to [8], wherein the fibrosis is cardiac fibrosis, preretinal fibrosis, gastrointestinal fibrosis, intestinal fibrosis, Crohn's disease, liver fibrosis, liver cirrhosis, chronic liver disease, scleroderma, idiopathic interstitial pneumonia, lung fibrosis, kidney fibrosis, nephrogenic systemic fibrosis, chronic kidney disease, chronic pancreatitis, cystic fibrosis, myelofibrosis, retroperitoneal fibrosis, mediastinal fibrosis, or joint fibrosis.

The present invention also includes the following embodiments.
[A1]
   A method for treating or preventing fibrosis, including administering a therapeutically effective amount of the compound according to any one of [1] to [5], or a pharmaceutically acceptable salt thereof, to a patient in need of treatment or prevention of fibrosis.
[A2]
   The method according to [A1], wherein the fibrosis is fibrosis in a heart, an eye, a brain, a digestive organ, a skin, a lung, a kidney, a hematopoietic organ, a retroperitoneum, a mediastinum, or a joint.
[A3]
   The pharmaceutical composition according to [A1] or [A2], wherein the fibrosis is cardiac fibrosis, preretinal fibrosis, gastrointestinal fibrosis, intestinal fibrosis, Crohn's disease, liver fibrosis, liver cirrhosis, chronic liver disease, scleroderma, idiopathic interstitial pneumonia, lung fibrosis, kidney fibrosis, nephrogenic systemic fibrosis, chronic kidney disease, chronic pancreatitis, cystic fibrosis, myelofibrosis, retroperitoneal fibrosis, mediastinal fibrosis, or joint fibrosis.

[B1] The compound according to any one of [1] to [5], or a pharmaceutically acceptable salt thereof, for use in treating or preventing fibrosis.
[B2] The compound according to [B1], or a pharmaceutically acceptable salt thereof, wherein the fibrosis is fibrosis in a heart, an eye, a brain, a digestive organ, a skin, a lung, a kidney, a hematopoietic organ, a retroperitoneum, a mediastinum, or a joint.
[B3] The compound according to [B1] or [B2], or a pharmaceutically acceptable salt thereof, wherein the fibrosis is cardiac fibrosis, preretinal fibrosis, gastrointestinal fibrosis, intestinal fibrosis, Crohn's disease, liver fibrosis, liver cirrhosis, chronic liver disease, scleroderma, idiopathic interstitial pneumonia, lung fibrosis, kidney fibrosis, nephrogenic systemic fibrosis, chronic kidney disease, chronic pancreatitis, cystic fibrosis, myelofibrosis, retroperitoneal fibrosis, mediastinal fibrosis, or joint fibrosis.

[C1] Use of the compound according to any one of [1] to [5], or a pharmaceutically acceptable salt thereof, for treating or preventing fibrosis.
[C2] The use according to [C1], wherein the fibrosis is fibrosis in a heart, an eye, a brain, a digestive organ, a skin, a lung, a kidney, a hematopoietic organ, a retroperitoneum, a mediastinum, or a joint.
[C3] The use according to [C1] or [C2], wherein the fibrosis is cardiac fibrosis, preretinal fibrosis, gastrointestinal fibrosis, intestinal fibrosis, Crohn's disease, liver fibrosis, liver cirrhosis, chronic liver disease, scleroderma, idiopathic interstitial pneumonia, lung fibrosis, kidney fibrosis, nephrogenic systemic fibrosis, chronic kidney disease, chronic pancreatitis, cystic fibrosis, myelofibrosis, retroperitoneal fibrosis, mediastinal fibrosis, or joint fibrosis.

[D1] Use of the compound according to any one of [1] to [5], or a pharmaceutically acceptable salt thereof, in the production of a pharmaceutical composition for treating or preventing fibrosis.
[D2] The use according to [D1], wherein the fibrosis is fibrosis in a heart, an eye, a brain, a digestive organ, a skin, a lung, a kidney, a hematopoietic organ, a retroperitoneum, a mediastinum, or a joint.
[D3] The use according to [D1] or [D2], wherein the fibrosis is cardiac fibrosis, preretinal fibrosis, gastrointestinal fibrosis, intestinal fibrosis, Crohn's disease, liver fibrosis, liver cirrhosis, chronic liver disease, scleroderma, idiopathic interstitial pneumonia, lung fibrosis, kidney fibrosis, nephrogenic systemic fibrosis, chronic kidney disease, chronic pancreatitis, cystic fibrosis, myelofibrosis, retroperitoneal fibrosis, mediastinal fibrosis, or joint fibrosis.

With regard to any of [1] to [9], [A1] to [A3], [B1] to [B3], [C1] to [C3], and [D1] to [D3], there may be a preferred aspect of any of the items described below as the present embodiment. In addition, with regard to any of [1] to [9], [A1] to [A3], [B1] to [B3], [C1] to [C3], and [D1] to [D3], a combination of any preferred aspects described below as the present embodiment may be selected. Preferred aspects include more preferred aspects, even more preferred aspects, and still more preferred aspects.

### Advantageous Effects of Invention

According to the present invention, a novel compound that can be used for the treatment or prevention of fibrosis can be provided.

### Brief Description of Drawings

[Figure 1]Fig. 1 shows the results of expression of marker genes for fibrosing after Compound (II) and Compound (III) were allowed to act on LX-2 cells. The error bars were calculated using Mean and SEM.
[Figure 2]Fig. 2 shows the results of immunostaining after Compound (II) was allowed to act on LX-2 cells.
[Figure 3]Fig. 3 shows the results of expression of marker genes for fibrosing after Compound (II) was allowed to act on quiescent hepatic stellate cell-like cells (qHSC-like cells) .
[Figure 4]Fig. 4 shows the results of measuring the range in which CollagenI of liver tissue sections is positive after Compound (II) was allowed to act on a mouse model of liver fibrosis.
[Figure 5]Fig. 5 shows the results of immunostaining of liver tissue sections after Compound (II) was allowed to act on a mouse model of liver fibrosis.
[Figure 6]Fig. 6 shows the results of expression of marker genes for fibrosing in liver tissue sections after Compound (II) was allowed to act on a mouse model of liver fibrosis.
[Figure 7]Fig. 7 shows the results of expression of marker genes for fibrosing after Compound (II) and Compound (III) were allowed to act on primary cultured human renal fibroblasts. The results are results of a group that was not stimulated with TGFβ1.
[Figure 8]Fig. 8 shows the results of expression of marker genes for fibrosing after Compound (II) and Compound (III) were allowed to act on primary cultured human renal fibroblasts. The results are results of a group that was stimulated with TGFβ1.
[Figure 9]Fig. 9 shows the results of expression of marker genes for fibrosing after Compound (II) and Compound (III) were allowed to act on primary cultured human lung fibroblasts. The results are results of a group that was not stimulated with TGFβ1.
[Figure 10]Fig. 10 shows the results of expression of marker genes for fibrosing after Compound (II) and Compound (III) were allowed to act on primary cultured human lung fibroblasts. The results are results of a group that was stimulated with TGFβ1.
[Figure 11]Fig. 11 shows the results of expression of marker genes for fibrosing after Compound (II) and Compound (III) were allowed to act on primary cultured human pancreatic fibroblasts. The results are results of a group that was not stimulated with TGFβ1.
[Figure 12]Fig. 12 shows the results of expression of marker genes for fibrosing after Compound (II) and Compound (III) were allowed to act on primary cultured human pancreatic fibroblasts. The results are results of a group that was stimulated with TGFβ1.
[Figure 13]Fig. 13 shows the results of expression of marker genes for fibrosing after Compound (II) and Compound (III) were allowed to act on primary cultured human skin fibroblasts. The results are results of a group that was not stimulated with TGFβ1.
[Figure 14]Fig. 14 shows the results of expression of marker genes for fibrosing after Compound (II) and Compound (III) were allowed to act on primary cultured human skin fibroblasts. The results are results of a group that was stimulated with TGFβ1.

### Description of Embodiments

Hereinafter, embodiments for carrying out the present invention will be described in detail. The present invention is not limited to the following embodiments, and various modifications can be made within the scope of the gist of the present invention.

### [Compound]

An embodiment of the present invention relates to a compound represented by the following Formula (I) or a pharmaceutically acceptable salt thereof. 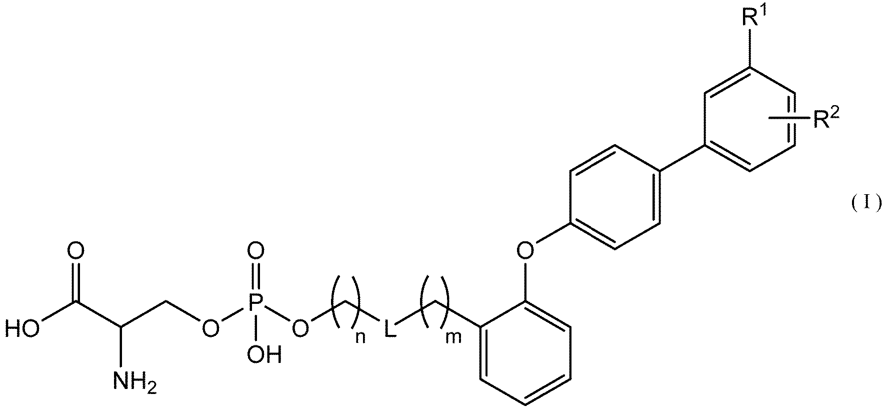 wherein in Formula (I),
L represents -O-, -NR-, -S-, -(C=O)O-, -O(C=O)-, - (C=O)NR-, -NR(C=O)-, -(C=S)NR-, -NR(C=S)-, -(C=O)S-, - S(C=O)-, -(C=S)O-, -O(C=S)-, (C=S) S-, or -S (C=S) -;
R represents a hydrogen atom or an aliphatic hydrocarbon group having 1 to 10 carbon atoms which may have a substituent;
R¹ and R² each independently represent an aliphatic hydrocarbon group having 1 to 10 carbon atoms which may have a substituent, an aromatic hydrocarbon group which may have a substituent, or -OR³;
R³ represents an aliphatic hydrocarbon group having 1 to 10 carbon atoms which may have a substituent, or an aromatic hydrocarbon group which may have a substituent; and
m and n are each independently an integer of 1 to 6.

In the present specification, the "aliphatic hydrocarbon group" means a non-aromatic group composed of carbon atoms and hydrogen atoms. The aliphatic hydrocarbon group is not particularly limited, and examples thereof include a chain-type hydrocarbon group and an alicyclic hydrocarbon group. The aliphatic hydrocarbon group may have one or more substituents.

In the present specification, the "substituent" is not particularly limited, and examples thereof include a hydroxy group (OH), an alkoxy group, a halogen atom (may be any of a fluorine atom, a chlorine atom, a bromine atom, or an iodine atom.), a cyano group, an amino group, a mono- or disubstituted amino group, a substituted silyl group, a nitro group, an azide group, an aromatic hydrocarbon group, a heteroaryl group, a heterocyclic group, an aliphatic hydrocarbon group, and an acyl group. When there are two or more substituents, they may be identical or different.

The substituent may be further substituted with a substituent. For example, the alkoxy group, aromatic hydrocarbon group, the heteroaryl group, the heterocyclic group, the aliphatic hydrocarbon group, the acyl group, and the like are substituents that may have a substituent.

In the present specification, the "chain-type hydrocarbon group" is not particularly limited, and examples thereof include alkyl, alkenyl, and alkynyl. The chain-type hydrocarbon group may be linear or branched.

In the present specification, the "alkyl" is not particularly limited, and examples thereof include methyl, ethyl, n-propyl, isopropyl, n-butyl, isobutyl, t-butyl, sec-butyl, n-pentyl, isopentyl, neopentyl, t-pentyl, 1,2-dimethylpropyl, n-hexyl, n-heptyl, isoheptyl, n-octyl, isooctyl, n-nonyl, and n-decyl.

In the present specification, the term "alkenyl" means a group having at least one double bond in alkyl. The alkenyl is not particularly limited, and examples thereof include vinyl, allyl, 1-propenyl, isopropenyl, 1-butenyl, 2-butenyl, 3-butenyl, 1,3-butanedienyl, 1-pentenyl, 2-pentenyl, 3-pentenyl, 4-pentenyl, 1,3-pentanedienyl, 1-hexenyl, 2-hexenyl, 3-hexenyl, 4-hexenyl, 5-hexenyl, and 1,4-hexanedienyl.

In the present specification, the term "alkynyl" means a group having at least one triple bond in alkyl. The alkynyl is not particularly limited, and examples thereof include ethynyl, 1-propynyl, 2-propynyl, butynyl, butadiynyl, pentynyl, pentadiynyl, hexynyl, and hexadiynyl.

In the present specification, the "alicyclic hydrocarbon group" is not particularly limited, and examples thereof include cycloalkyl and cycloalkenyl. The alicyclic hydrocarbon group may be monocyclic, bicyclic, tricyclic, or polycyclic.

In the present specification, the "cycloalkyl" is not particularly limited, and examples thereof include cyclopropyl, cyclobutyl, cyclopentyl, cyclohexyl, cycloheptyl, cyclooctyl, bicyclo[4.4.0]decanyl, bicyclo[1.1.1]pentyl, spiro[2.2]pentyl, bicyclo[2.2.1]heptyl, spiro[3.3]heptyl, bicyclo[2.2.2]octyl, and bicyclo[3.2.1]octyl.

In the present specification, the term "cycloalkenyl" means a group having at least one double bond in cycloalkyl. The cycloalkenyl is not particularly limited, and examples thereof include 1-cyclopentenyl, 1-cyclohexenyl, 1-cycloheptenyl, 1-cyclooctenyl, cyclopentadienyl, cyclohexadienyl, cycloheptadienyl, and cyclooctadienyl.

In the present specification, the aliphatic hydrocarbon group having a substituent is not particularly limited, and examples thereof include an aliphatic hydrocarbon group substituted with an aromatic hydrocarbon group which may have a substituent. The aliphatic hydrocarbon group substituted with an aromatic hydrocarbon group may be a group called aralkyl, and is not particularly limited, and examples thereof include benzyl, phenethyl, naphthylmethyl, 9-fluorenylmethyl, and triphenylmethyl.

In the present specification, the term "aromatic hydrocarbon group" means an aromatic group composed of carbon atoms and hydrogen atoms. The aromatic hydrocarbon group may be monocyclic, bicyclic, tricyclic, or polycyclic. The aromatic hydrocarbon group may have one or more substituents. The aromatic hydrocarbon group is not particularly limited, and examples thereof include phenyl, 1-naphthyl, 2-naphthyl, and anthryl.

In the present specification, the aromatic hydrocarbon group having a substituent is not particularly limited, and examples thereof include an aromatic hydrocarbon group substituted with an aliphatic hydrocarbon group which may have a substituent. The aromatic hydrocarbon group substituted with an aliphatic hydrocarbon group is not particularly limited, and examples thereof include methylphenyl and t-butylphenyl.

In Formula (I) of the present embodiment, L represents -O-, -NR-, -S-, -(C=O)O-, -O(C=O)-, -(C=O)NR-, - NR(C=O)-, -(C=S)NR-, -NR(C=S)-, -(C=O)S-, -S(C=O)-, - (C=S)O-, -O(C=S)-, (C=S)S-, or -S(C=S)-, and among these, from the viewpoint of further exerting the effect of treating or preventing fibrosis, -(C=O)O-, -O(C=O)-, - (C=O)NR-, -NR(C=O)-, -(C=S)NR-, -NR(C=S)-, -(C=O)S-, - S(C=O)-, -(C=S)O-, -O(C=S)-, (C=S)S-, or -S(C=S)- is preferable, while -(C=O)O-, -O(C=O)-, -(C=O)NR-, or - NR(C=O)- is more preferable.

In Formula (I) of the present embodiment, R represents a hydrogen atom or an aliphatic hydrocarbon group having 1 to 10 carbon atoms which may have a substituent, and among these, from the viewpoint of further exerting the effect of treating or preventing fibrosis, R is preferably a hydrogen atom or an aliphatic hydrocarbon group having 1 to 5 carbon atoms which may have a substituent, and more preferably a hydrogen atom.

In Formula (I) of the present embodiment, when L is -NR-, -(C=O)NR-, -NR(C=O)-, -(C=S)NR-, or -NR(C=S)-, L is preferably -NH-, -(C=O)NH-, -NH(C=O)-, -(C=S)NH-, or - NH(C=S)-.

In Formula (I) of the present embodiment, R¹ and R² each independently represent an aliphatic hydrocarbon group having 1 to 10 carbon atoms which may have a substituent, an aromatic hydrocarbon group which may have a substituent, or -OR³, and from the viewpoint of further exerting the effect of treating or preventing fibrosis, R¹ and R² are preferably each independently an aliphatic hydrocarbon group having 1 to 7 carbon atoms which may have a substituent, an aromatic hydrocarbon group which may have a substituent, or -OR³; more preferably each independently ethyl, isopropyl, t-butyl, phenyl, methylphenyl, benzyl, trifluoromethyl, or methoxy; and even more preferably isopropyl, t-butyl, phenyl, or methylphenyl, while R¹ and R² may be each isopropyl, t-butyl, or phenyl, or may be t-butyl or phenyl.

In Formula (I) of the present embodiment, R³ represents an aliphatic hydrocarbon group having 1 to 10 carbon atoms which may have a substituent, or an aromatic hydrocarbon group which may have a substituent, and among these, from the viewpoint of further exerting the effect of treating or preventing fibrosis, R³ is preferably an alkyl having 1 to 5 carbon atoms which may have a substituent, an alkenyl having 2 to 5 carbon atoms, an alkynyl having 2 to 5 carbon atoms, or an aromatic hydrocarbon group which may have a substituent, and more preferably methyl, ethyl, vinyl, allyl, ethynyl, phenyl, methylphenyl, or benzyl. For example, when R³ is methyl, -OR³ can be expressed differently as methoxy, and when R³ is phenyl, -OR³ can be expressed differently as phenoxy.

In Formula (I) of the present embodiment, R¹ and R² may be each independently any group independently selected from the preferred groups, more preferred groups, and even more preferred groups described for each, and R¹ and R² may be any combination of groups shown in the present specification. For example, a preferred group may be selected as R¹, and a more preferred group may be selected as R². R¹ and R² may be identical, and in that case, R¹ and R² may be a preferred group described for each, may be a more preferred group, or may be an even more preferred group.

In Formula (I) of the present embodiment, R¹ and R² may be different or identical; however, from the viewpoint of further exerting the effect of treating or preventing fibrosis and from the viewpoint of ease of synthesis, it is preferable that R¹ and R² are identical, and R¹ and R² are each more preferably ethyl, isopropyl, t-butyl, phenyl, methylphenyl, benzyl, trifluoromethyl, or methoxy, and even more preferably isopropyl, t-butyl, phenyl, or methylphenyl, while R¹ and R² may be isopropyl, t-butyl, or phenyl, or may be t-butyl or phenyl. When R¹ and R² are each independently -OR³, the condition that R¹ and R² are different means that R³ is different, and the condition that R¹ and R² are identical means that R³ is identical.

In Formula (I) of the present embodiment, the bonding position of R² is not particularly limited; however, from the viewpoint of further exerting the effect of treating or preventing fibrosis, it is preferable that R² is located at the meta-position or the ortho-position, and more preferably located at the meta-position, with respect to the bonding position of R¹.

When R² is located at the meta-position with respect to the bonding position of R¹, it means that R¹ and R² are 3,5-substituted in the benzene ring to be bonded.

Examples of the case where R² is located at the meta-position with respect to the bonding position of R¹ include, but are not particularly limited to, compounds represented by Formula (II), Formula (III), Formula (XI), or Formula (XII).

In Formula (I) of the present embodiment, m and n are each independently an integer of 1 to 6, preferably each independently an integer of 1 to 4, and preferably each independently an integer of 2 to 3. The sum of m and n (m+n) is an integer of 2 to 12, preferably an integer of 3 to 8, and more preferably 3 to 6.

In Formula (I) of the present embodiment, m and n may be each independently the preferable integer, may be the more preferable integer, or may be the even more preferable integer, described for each. In addition, m and n may be any combination of integers independently selected from the preferable integers, the more preferable integers, and the even more preferable integers, described for each. For example, a preferable integer may be selected as m, and a more preferable integer may be selected as n.

In the present embodiment, the compound represented by Formula (I) is not particularly limited; however, for example, the compound is preferably a compound represented by any one of the following Formula (IV) to the following Formula (VII), and more preferably a compound represented by the following Formula (IV) or the following Formula (V): 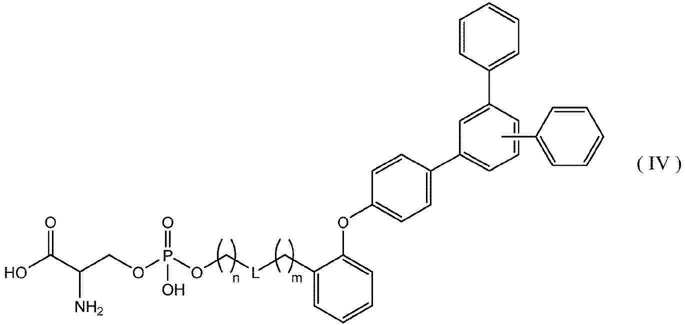 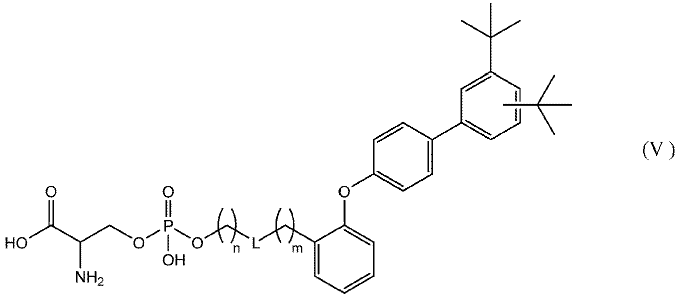 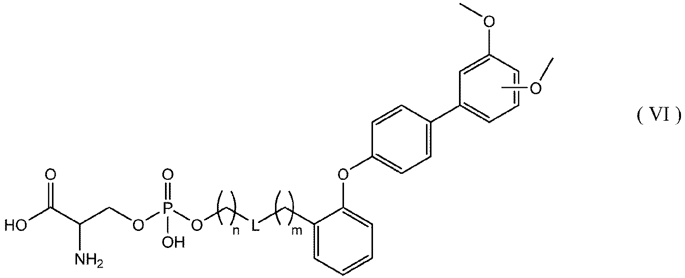 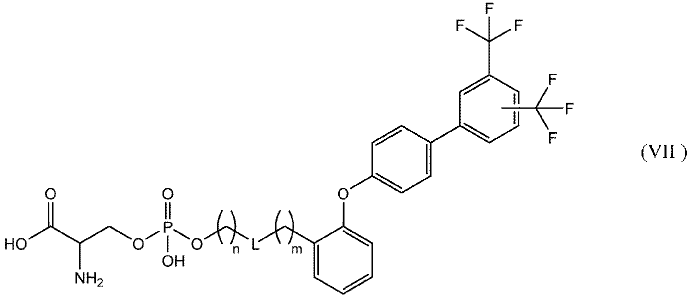 wherein in Formula (IV) to Formula (VII), L, m, and n each have the same meaning as the definition in Formula (I), and may be an aspect described as a preferred aspect, or any combination can be selected.

In the present embodiment, the compound represented by Formula (I) is not particularly limited, but is preferably, for example, a compound represented by the following Formula (VIII) or the following Formula (IX): 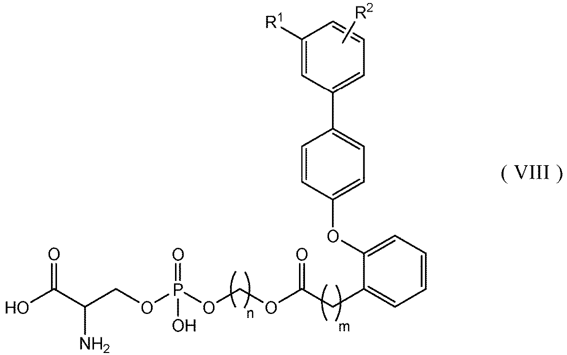 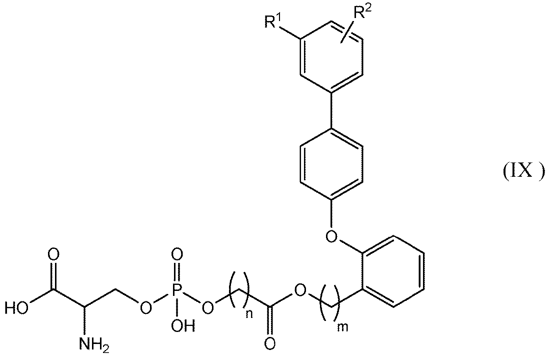 wherein in Formula (VIII) or Formula (IX), R¹, R², m, and n each have the same meaning as the definition in Formula (I), and may be an aspect described as a preferred aspect, or any combination can be selected.

Incidentally, when R¹ and R² each have the same meaning as the definition in Formula (I), it means that R³ in a case where R¹ and R² are each independently -OR³ also has the same meaning as the definition in Formula (I). Hereinafter, the same applies throughout the present specification.

In Formula (VIII) or Formula (IX), R¹ and R² are preferably in a relationship in which R² is located at the meta-position with respect to the bonding position of R¹, and R¹ and R² are more preferably in a relationship in which R² is located at the meta-position with respect to the bonding position of R¹, while R¹ and R² are identical.

In addition, in Formula (VIII) or Formula (IX), the structure of the benzene ring to which R¹ and R² are bonded is preferably a structure of any of Formula (IV) to Formula (VII). In this case, R¹ and R² are preferably in a relationship in which R² is located at the meta-position with respect to the bonding position of R¹. It is more preferable that R¹ and R² are in a relationship in which R² is located at the meta-position with respect to the bonding position of R¹, and m and n each have the same meaning as the definition in Formula (I) and are aspects described as preferred aspects.

In the present embodiment, the compound represented by Formula (I) is not particularly limited but is preferably a compound represented by the following Formula (X) : 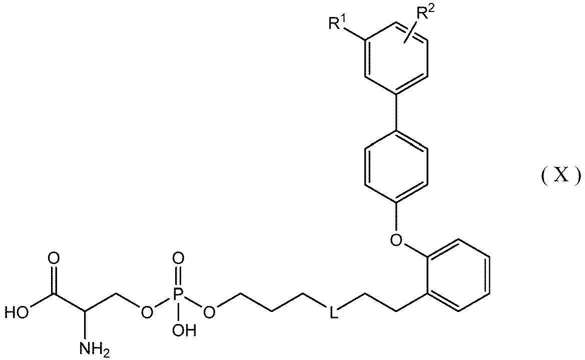 wherein in Formula (X), R¹, R², and L each have the same meaning as the definition in Formula (I) and may be an aspect described as a preferred aspect, and any combination can be selected.

In Formula (X), R¹ and R² are preferably in a relationship in which R² is located at the meta-position with respect to the bonding position of R¹, and R¹ and R² are more preferably in a relationship in which R² is located at the meta-position with respect to the bonding position of R¹, while R¹ and R² are identical.

In addition, in Formula (X), the structure of the benzene ring to which R¹ and R² are bonded is preferably a structure of any of Formula (IV) to Formula (VII). In this case, R¹ and R² are preferably in a relationship in which R² is located at the meta-position with respect to the bonding position of R¹. It is more preferable that R¹ and R² are in a relationship in which R² is located at the meta-position with respect to the bonding position of R¹, and L has the same meaning as the definition in Formula (I) and is an aspect described as a preferred aspect.

In the present embodiment, the compound represented by Formula (I) is not particularly limited but is preferably a compound represented by the following Formula (II), the following Formula (III), the following Formula (XI), or the following Formula (XII).

In addition, in Formula (I) of the present embodiment, R¹ and R² are not particularly limited from the viewpoint of further exerting the effect of treating or preventing fibrosis, but are each independently preferably a hydrophobic group or/and a high-shade group, and the compound represented by Formula (I) is more preferably a compound represented by the following Formula (II) or the following Formula (III).

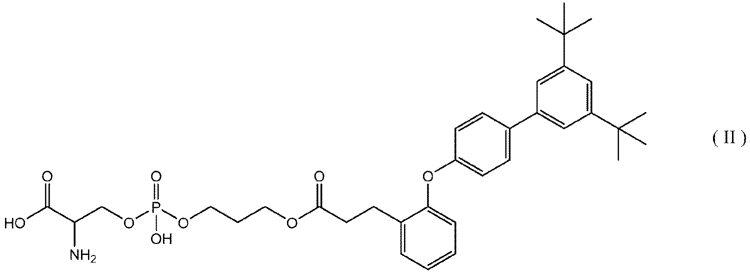

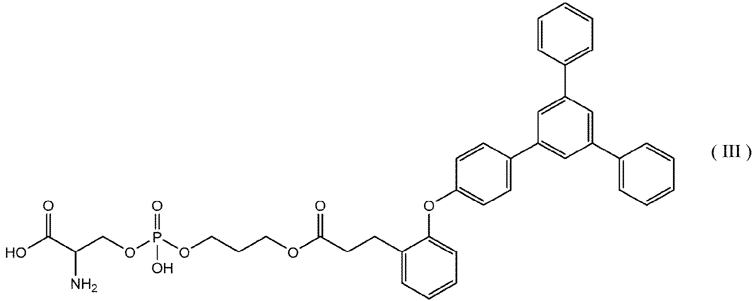

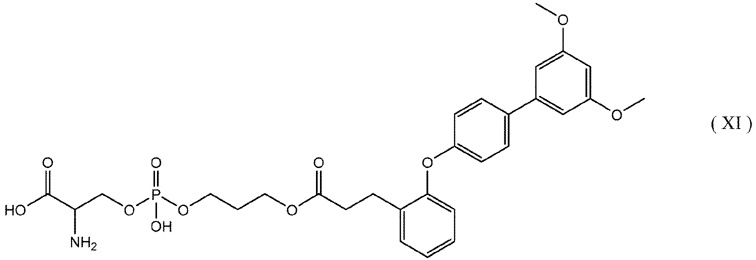

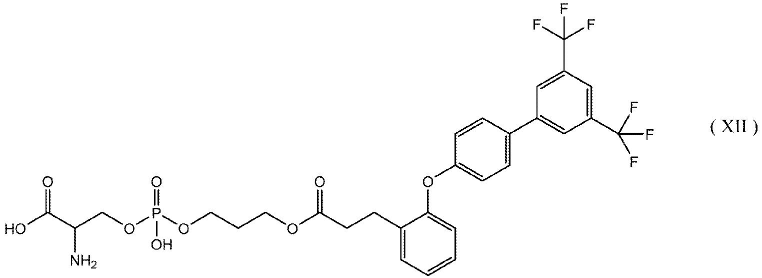

In Formula (II), Formula (III), Formula (XI), or Formula (XII), the structure of L in the compound represented by Formula (I) is described as -O(C=O)-; however, the structure described as -O(C=O)- may have the same meaning as the definition of L in Formula (I). That is, the structure described as -O(C=O)- in Formula (II), Formula (III), Formula (XI), or Formula (XII) may be L, or may be an embodiment of L described as a preferred embodiment. Above all, the structure described as -O(C=O)-in Formula (II), Formula (III), Formula (XI), or Formula (XII) may be -(C=O)O-.

In the present specification, the compound represented by Formula (II), Formula (III), Formula (XI), or Formula (XII) may be described as Compound (II), Compound (III), Compound (XI), or Compound (XII), respectively.

In the present embodiment, the compound represented by the above Formula (I) may be a pharmaceutically acceptable salt thereof. The pharmaceutically acceptable salt is not particularly limited, and examples thereof include a base addition salt, an acid addition salt, and an amino acid addition salt. The base addition salt is not particularly limited, and examples thereof include metal salts such as a sodium salt, a potassium salt, a calcium salt, and a magnesium salt; ammonium salts; or organic amine salts such as a triethylamine salt, a piperidine salt, and a morpholine salt. The acid addition salt is not particularly limited, and examples thereof include mineral acid salts such as a hydrochloric acid salt, a sulfuric acid salt, and a nitric acid salt; and organic acid salts such as a methanesulfonic acid salt, a para-toluenesulfonic acid salt, a citric acid salt, and an oxalic acid salt. The amino acid addition salt is not particularly limited, and examples thereof include glycine salts.

The carboxylic acid, the amino group, the phosphoric acid, or the like in Formula (I) of the present embodiment may form a pharmaceutically acceptable salt or may exist in a free state. Furthermore, Formula (I) of the present embodiment may have a partial structure having a positive charge or a negative charge or may have a partial structure forming a cation or an anion, and the charge of those partial structures may be changed. When the compound represented by Formula (I) of the present embodiment is present in aqueous solutions having various pH values or has a crystalline structure, the compound may have a partial structure that forms a cation or an anion, or the aqueous solution or the like may be adjusted so that the compound represented by Formula (I) has a partial structure that forms a cation or an anion. Specific examples of such a partial structure include, but are not particularly limited to, -(C=O)O⁻, -NH₃⁺, and -O(C=P)(O⁻)O-. In addition, when a partial structure forming a cation or an anion is present in Formula (I) of the present embodiment, a hydrogen bond, an ionic bond, or the like may be formed between the partial structures. Furthermore, the hydrogen bond or the ionic bond may be an intramolecular bond or an intermolecular bond, and when the hydrogen bond or the ionic bond is an intramolecular bond, a cyclic structure may be formed in the molecule by the hydrogen bond or the ionic bond.

The above-described compound represented by Formula (I) or a pharmaceutically acceptable salt thereof may exist as a hydrate or a solvate; however, all of these substances are included in the scope of the present invention. The type of the solvent forming the solvate is not particularly limited, and examples thereof include ethanol, acetone, and isopropanol.

When there are stereoisomers (for example, enantiomers and diastereomers) of the above-described compound represented by Formula (I) and the pharmaceutically acceptable salt thereof, the individual stereoisomers and mixtures thereof (for example, racemates) are included in the compound represented by Formula (I) and the pharmaceutically acceptable salt thereof.

### [Method for Producing Compound]

The above-described compound represented by Formula (I) is not particularly limited but can be produced, for example, according to the methods of the following Schemes 1 to 3. In Compound A to Compound I, L, R¹, R², m, and n have the same meanings as the definitions in the above Formula (I), respectively.

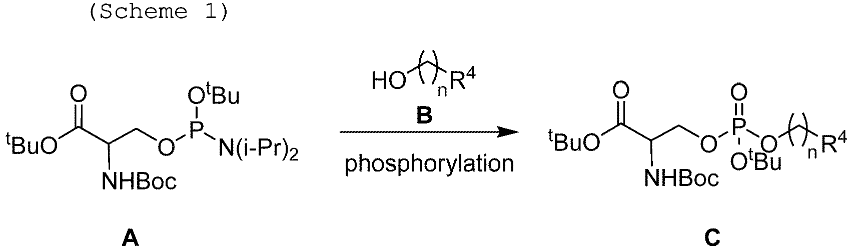

By allowing a compound B to act on a known compound A, a phosphoric acid group is formed, and a compound C is obtained. In the compound B, R⁴ is not particularly limited but represents a leaving group such as a halogen atom, -OR^{4'}, -N(R^{4'})₂, -SR^{4'}, -(C=O)OR^{4'}, -(C=O)NH₂, - (C=S)N(R^{4'})₂, -(C=O)SR^{4'}, -(C=S)OR^{4'}, or -(C=S)SR^{4'}. R^{4'} may be a hydrogen atom or a protective group. When R^{4'} is a protective group, a deprotection reaction may be performed after the above-described reaction.

As the protective group, known protective groups can be each utilized, and for example, a protective group described in Greene's Protective Groups in Organic Synthesis can be selected.

In addition, by using an optically active compound A, an optically active compound represented by the above Formula (I) can be synthesized.

More specifically, the compound C can be synthesized, for example, by the method described in Ikubo M., et al, J. Med. Chem. 2015, 58, 4204-4219.

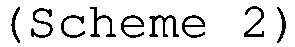

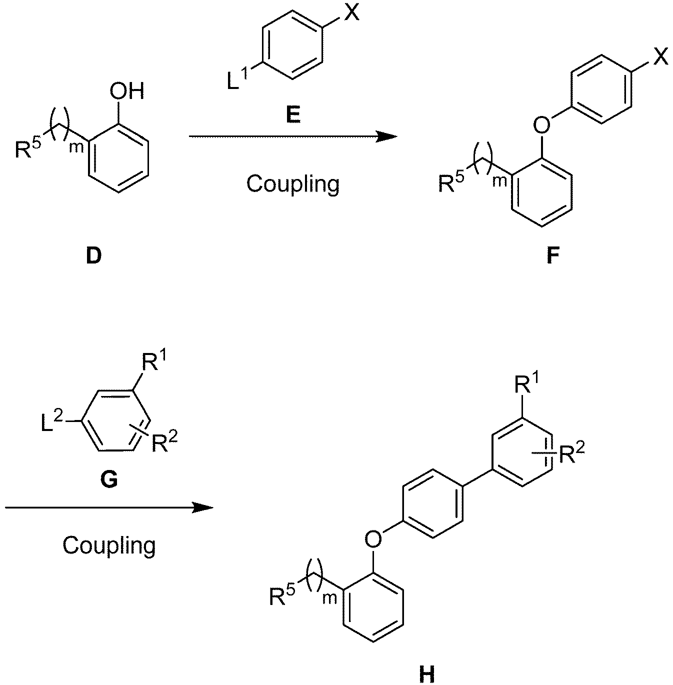

A compound F is obtained by a coupling reaction between a known compound D and a compound E.

In the compound D, R⁵ is not particularly limited but represents, for example, a leaving group such as a halogen atom, -OR^{5'}, -N(R^{5'})₂, -SR^{5'}, -(C=O)OR^{5'}, -(C=O)NH₂, - (C=S)N(R^{5'})₂, -(C=O)SR^{5'}, -(C=S)OR^{5'}, or -(C=S)SR^{5'}. R^{5'} may be a hydrogen atom or a protective group. When R^{5'} is a protective group, a deprotection reaction may be performed after the above reaction, and the protective group is not particularly limited; however, examples thereof include the above-described protective groups.

In the compound E, L¹ is preferably a functional group capable of undergoing a coupling reaction with a hydroxy group in the compound D. L¹ is not particularly limited, and examples thereof include boronic acid. The coupling reaction is not particularly limited, and examples thereof include a coupling reaction using a boron compound, a coupling reaction using an organolithium compound, a coupling reaction using an organozinc compound, a coupling reaction using a tin compound, and an aromatic nucleophilic substitution reaction, while a coupling reaction known in the field of synthetic organic chemistry can be appropriately used. More specific examples thereof include a Suzuki-Miyaura coupling reaction, a Negishi coupling reaction, and a Stille coupling reaction.

In the compound E, X is preferably a functional group capable of undergoing a coupling reaction with L² in a compound G. X is not particularly limited, and examples thereof include a halogen atom and a sulfonyl group. Examples of the coupling reaction include the above-described reactions, and a coupling reaction known in the field of organic synthetic chemistry can be appropriately used.

Furthermore, X may be converted to a group preferable for a coupling reaction with the compound G by converting the obtained compound F.

A compound H is obtained by a coupling reaction between the obtained compound F and a compound G.

In the compound G, L² is preferably a functional group capable of undergoing a coupling reaction with X in the compound F. L² is not particularly limited, and examples thereof include boronic acid. Examples of the coupling reaction include the above-described reactions, and a coupling reaction known in the field of organic synthetic chemistry can be appropriately used.

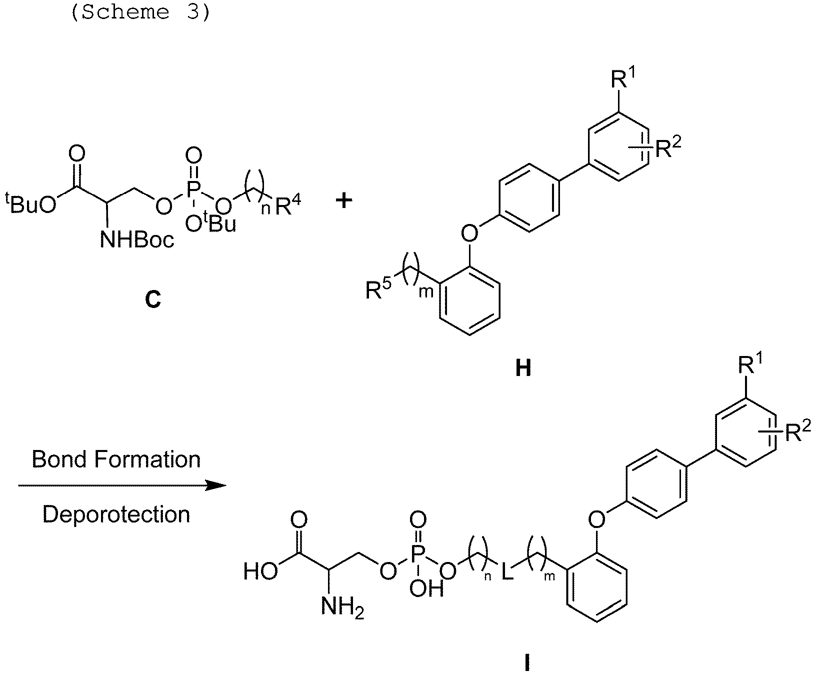

A compound I is obtained by a bond formation reaction and a deprotection reaction between the obtained compound C and a compound H.

Examples of the bond formation reaction include a bond formation reaction using a condensing agent, an S_{N}2 nucleophilic substitution reaction, an ester bond formation reaction using an acid or a base, and an amide bond formation reaction, and a bond formation reaction known in the field of synthetic organic chemistry can be appropriately used. More specific examples include an ester bond formation reaction using a condensing agent such as N,N'-dicyclohexylcarbodiimide, an amide bond formation reaction, an ester bond formation reaction by a Fisher method, and a Williamson ether synthesis reaction.

Furthermore, after the compound I in which L has a carbonyl group is obtained, a target compound can be obtained by converting the carbonyl group to a thiocarbonyl group or the like.

As the deprotection reaction, a known deprotection reaction can be utilized, and for example, a reaction described in Greene's Protective Groups in Organic Synthesis can be selected.

The solvent and the catalyst used in each step of the production method, the equivalents of reagents, and each of reaction conditions such as a reaction temperature and a reaction time will be described in detail as representative examples in the Examples that will be described below but are not necessarily limited thereto, and those skilled in the art can appropriately select each of the conditions based on general knowledge in organic synthesis.

### [Pharmaceutical Composition and Treatment and/or Prevention]

An embodiment of the present invention relates to a pharmaceutical composition containing any one compound of the compounds represented by Formula (I) to Formula (XII) described above, or a pharmaceutically acceptable salt thereof. An embodiment of the present invention also relates to a pharmaceutical composition for treating or preventing a disease, containing a compound represented by the above Formula (I) or a pharmaceutically acceptable salt thereof.

In the present specification, the term "treatment or prevention" includes therapeutic and/or prophylactic treatment. The term "therapeutic or prophylactic" treatment includes administration of a pharmaceutical composition to a subject, which has been proved in the pertinent art. When administered prior to a clinical manifestation of an undesirable condition (for example, a disease or other undesirable condition of the subject), the treatment is prophylactic (that is, the subject is protected from the onset of an undesirable condition), whereas when administered after a manifestation of an undesirable condition, the treatment is therapeutic (that is, it is intended to diminish, suppress, ameliorate, or stabilize an existing undesirable state or side effects thereof).

In the present specification, the term "pharmaceutical composition" encompasses not only products including an active ingredient and an inert ingredient that constitutes a carrier (such as a pharmaceutically acceptable excipient or additive), but also any products that occur directly or indirectly as a result of association, complexation, or aggregation of any two or more components, or as a result of dissociation of one or more components, or as a result of another type of reaction or interaction of one or more components.

In the present embodiment, the active ingredient may be a compound represented by the above Formula (I) or a pharmaceutically acceptable salt thereof.

When the pharmaceutical composition is administered as a therapeutic agent, the subject to be administered is a mammal. Examples of the mammal include, but are not particularly limited to, humans, non-human primates, domestic animals, laboratory animals, and livestock, and the mammal is preferably human.

When the pharmaceutical composition is administered as a therapeutic agent, there is no particular limitation; however, for example, the pharmaceutical composition may be orally administered as a tablet preparation, a powder preparation, a granular preparation, a capsule preparation, or a syrup preparation, or may be parenterally administered as an injectable preparation or a drip infusion. The administration route as an injectable preparation or a drip infusion is not particularly limited but may be, for example, subcutaneous administration or intravenous administration. Furthermore, the pharmaceutical composition may also be administered as a patch or the like.

When formulating a pharmaceutical composition, a pharmaceutical composition in a desired dosage form can be produced by a conventional method using an ordinary carrier. In the case of preparing an oral preparation, a conventionally known method for preparing an oral solid preparation can be applied, an excipient and, if necessary, additives such as a binder, a disintegrant, a stabilizer, and a lubricant are added to the active ingredient, and then the mixture may be prepared into a tablet preparation, a powder preparation, a granular preparation, or the like by a conventional method. A capsule preparation and a syrup preparation can be prepared by conventional methods. Furthermore, the syrup preparation may be of a prior preparation type. In the case of preparing an injectable preparation, a pH adjusting agent, a buffering agent, a stabilizing agent, a solubilizing agent, and the like may be added to the principal agent as necessary, and the mixture may be formed into an injectable preparation, a drip infusion, or the like by a conventional method. The preparation of the patch is not particularly limited, and the patch can be prepared by a conventional method.

The dosage and administration interval of the active ingredient of the pharmaceutical composition can be appropriately selected according to the subject to be administered, the route of administration, the disease, and the age, body weight, and symptoms of the subject. For example, in the case of oral administration, there is no particular limitation; however, for example, for an adult, the dose of the active ingredient is 0.01 mg to 10 g, may be 100 mg to 6 g, or may be 50 mg to 500 mg, per day. Regarding the administration interval of the pharmaceutical composition, a daily dosage may be administered once a day, or may be administered in several divided doses.

### (Fibrosis)

The pharmaceutical composition of the present embodiment is preferably used for the treatment or prevention of fibrosis.

Fibrosis is a disease that occurs in various organs and tissues, and is known to be an underlying disease that changes over to liver cirrhosis, renal failure, heart failure, pancreatic cancer, or the like and includes fibrosing in organs and tissues.

Fibrosing is one of the wound healing processes, but excessive fibrosing impairs the original functions of organs and tissues and progresses to pathogenesis. Particularly, fibrosing such as liver cirrhosis, scleroderma or idiopathic pneumonia significantly deteriorates the prognosis of patients and is life-threatening.

Here, the compound represented by the above Formula (I) in the present embodiment provides a novel therapeutic method or a novel prophylactic method, which is effective against this fibrosing by a deactivating action on activated cells that contribute to fibrosing. The compound represented by Formula (I) may be a compound represented by any one of the above Formula (II) to Formula (XII), or may be a pharmaceutically acceptable salt of a compound represented by any one of the above Formula (I) to Formula (XII). Hereinafter, the same applies to the description of the compound represented by Formula (I).

The organ and tissue is not particularly limited, and examples thereof include a heart, an eye, a brain, a digestive organ, a skin, a lung, a kidney, a hematopoietic organ, a retroperitoneum, a mediastinum, and a joint. The fibrosis in such an organ is not particularly limited, and examples thereof include cardiac fibrosis, preretinal fibrosis, gastrointestinal fibrosis, intestinal fibrosis, Crohn's disease, liver fibrosis, liver cirrhosis, chronic liver disease, scleroderma, idiopathic interstitial pneumonia, lung fibrosis, kidney fibrosis, nephrogenic systemic fibrosis, chronic kidney disease, chronic pancreatitis, cystic fibrosis, myelofibrosis, retroperitoneal fibrosis, mediastinal fibrosis, and joint fibrosis. Scleroderma includes systemic scleroderma and localized scleroderma.

The fibrosis is preferably fibrosis in the digestive organ, the skin, the lung, or the kidney, more preferably fibrosis in the pancreas, the skin, the lung, or the kidney, and even more preferably liver fibrosis, liver cirrhosis, chronic liver disease, and the like.

Among them, hepatic fibrosing caused by hepatitis virus, non-alcoholic steatohepatitis (NASH), non-alcoholic fatty liver disease (NAFLD), and the like, which are drastically increased due to lifestyle, progresses to liver cirrhosis, for which there is no effective treatment method other than transplantation. Since improvement of liver fibrosing also improves the liver function, the compound represented by the above Formula (I) in the present embodiment can be used for the treatment or prevention of liver fibrosis.

The compound represented by the above Formula (I) in the present embodiment can also be used for the treatment or prevention of liver fibrosing associated with NASH, hepatitis B virus (HBV) infection, excessive intake of alcohol, or the like.

Furthermore, regarding systemic scleroderma designated as an incurable disease, the compound represented by the above Formula (I) in the present embodiment can provide an effective therapeutic or prophylactic method. In addition, also regarding interstitial lung fibrosis designated as an incurable disease, the compound represented by the above Formula (I) in the present embodiment also contributes to overcoming such an incurable disease as a novel therapeutic agent or prophylactic agent for fibrosis. Furthermore, the compound represented by the above Formula (I) in the present embodiment can also be an effective therapeutic agent or prophylactic agent for fibrosis in the heart, kidney, pancreas, intestine, and the like.

Suppression of activation and deactivation in fibroblasts or fibroblast-like cells (myofibroblasts) such as hepatic stellate cells have been reported to be effective for the treatment of fibrosis.

In the present embodiment, as the compound represented by the above Formula (I) acts on cells in an activated state of fibroblast-like cells (myofibroblasts) to promote deactivation, the compound can be used for treatment or prevention of fibrosis.

In an organ in which fibrosis has regressed, it is known that fibroblast-like cells (myofibroblasts) undergo apoptosis or enter a deactivated cell state like a quiescent state. Deactivated fibroblast-like cells (myofibroblasts) stop the production and proliferation of fibers, express environmental factors of cells such as pleiotrophin and midkine, and contribute not only to improvement of fibrosis but also to normalization of organs and tissues. In a mouse liver fibrosis model, it has been shown that when the transcription factor TCF21 is introduced into activated hepatic stellate cells, which are fibroblast-like cells, deactivation of the hepatic stellate cells is promoted, fibrosis is improved, and the liver function is also improved.

With regard to the treatment or prevention of fibrosis based on the deactivating action in the present embodiment, for example, the following documents can be referred to. The compound represented by the above Formula (I) in the present embodiment also exhibits a therapeutic or prophylactic effect in a model animal of fibrosis.
(1) Kisseleva, T. Nature Reviews Gastroenterology & Hepatology, 2021, 18, 151-166.
(2) Jun J.-II, J Clin Invest. 2018, 128, 97-107.
(3) Zisser, A. Biomedicines, 2021, 9, 365.
(4) Ueha, S. Front. Immunol., 2012, 3, 71.
(5) Yazdani, S. Advanced Drug Delivery Reviews, 2017, 121, 101-116.
(6) Junien, J.L. Hepatology Communications 2017, 1, 524-537.
(7) Bellusci, S. Cell Stem Cell 2017, 21, 166-177.
(8) Lafyatis, R. Nature Reviews Rheumatology, 2019, 15, 705-730.
(9) Wynn, T.A. Nature, 2022, 587, 555-566.
(10) Radstake, T. Nature Reviews Rheumatology, 2018, 14, 657-673.
(11) Varga, J. Nature Reviews Rheumatology, 2019, 15, 208-224.
(12) Reilly, O. Nature Reviews Rheumatology, 2021, 17, 596-697.
(13) Horton, M.R. J. Clin. Invest. 2021:131, e143226.
(14) Selman, M. Nature Reviews Drug Discovery, 2017, 16, 755-772.
(15) Wells, A.U. Nature Reviews Disease Primers, 2017, 3, 1-19.
(16) Nakano, Y. Hepatology, 2020, 71, 1437-1452.

As an index of deactivation of fibroblasts or fibroblast-like cells (myofibroblasts) such as hepatic stellate cells, a known fibrosing-associated gene marker can be used as an index. The fibrosing-associated gene marker is not particularly limited, and for example, ACTA2(αSMA), COL1A1, COL3A1, and the like (in the present specification, they may be collectively referred to as "marker gene for fibrosing") are known. "ACTA2(αSMA)" is α-smooth muscle actin 2, "COL1A1" is an abbreviation for Collagen, Type I, Alpha 1 (type I collagen α1), and "COL3A1" is an abbreviation for Collagen, Type III, Alpha 1 (type III collagen α1).

Here, reduction in the expression of the marker gene for fibrosing means that activated cells in the fibroblast-like cells (myofibroblasts) are deactivated.

### (Combined Administration)

As an embodiment of the present invention, the compound represented by the above Formula (I) may be administered in combination with another active ingredient.

The other active ingredient to be administered in combination with the compound represented by the above Formula (I) is not particularly limited, and may be a component known to be possibly involved in the treatment or prevention of fibrosis, or a component known to be possibly involved in the treatment or prevention of the above-described various diseases explained as fibrosis.

In the combined administration, the compound represented by the above Formula (I) and the other active ingredient may be administered at separate times.

When administered at the same time, the compound represented by the above Formula (I) and the other active ingredient may be contained in the same preparation, or may be administered as separate preparations.

When administered at separate times, the compound represented by the above Formula (I) and the other active ingredient may be administered according to a desired administration regimen. The dosage and administration interval of the other active ingredient may be in accordance with a predetermined administration regimen.

### (Combination Product)

An embodiment of the present invention may be a combination product for the treatment or prevention of fibrosis, including the compound represented by the above Formula (I) in the present embodiment and another active ingredient. A combination product generally refers to a therapeutic and diagnostic product combining agents (active components and active ingredients), devices, and/or biological products, and is more particularly defined by the United States Food and Drug Administration (FDA). For example, the FDA website <URL:https://www.fda.gov/combination-products> or the like can be referred to.

The combination product may be a kit including at least the compound represented by the above Formula (I) in the present embodiment and another active ingredient. Instructions may be attached to the kit. Regarding the instructions, information regarding medication instruction may be described therein.

### (Inhibitor of Fibrosing)

An embodiment of the present invention may be an inhibitor of fibrosing, containing the compound represented by the above Formula (I) or a pharmaceutically acceptable salt thereof. Since the compound represented by the above Formula (I) or a pharmaceutically acceptable salt thereof has a fibrosing inhibitory effect, the inhibitor of fibrosing may be used for the treatment or prevention of fibrosis.

### Examples

Hereinafter, the present embodiment will be described more specifically by way of Examples and Comparative Examples; however, the present embodiment is not limited only to these Examples. The measurement method used in the present embodiment is as follows.

[¹H, ¹³C, ³¹P-NMR]

Bruker Advance 400
[Mass Spectrometry]
Bruker micrOTOF-05 (ESI-TOF mass spectrometer)

### <Example 1>

### Synthesis of Compound (II)

### (Step 1) Synthesis of Compound 5

Compound 5 was synthesized according to the following reaction formula.

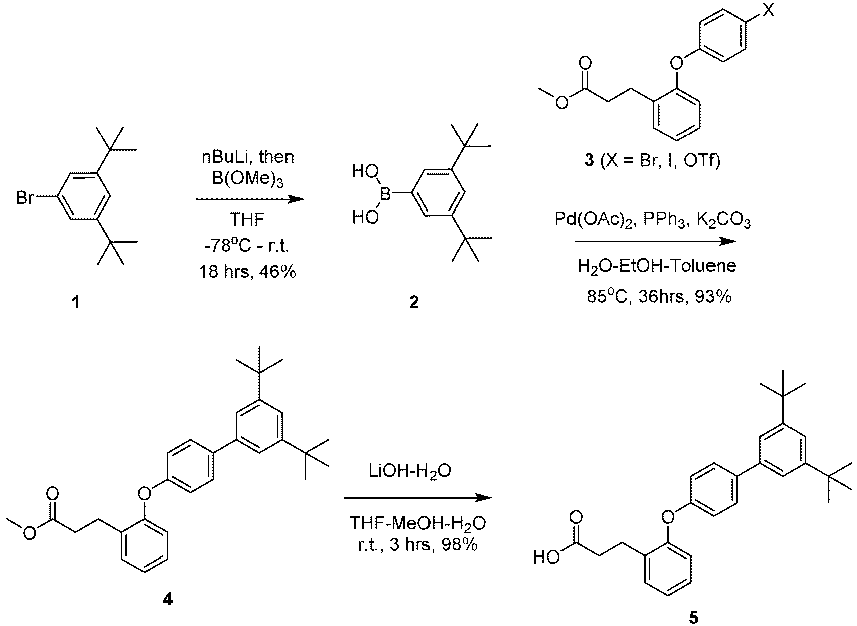

To a solution of Compound 1 (7.259 g, 27.080 mmol) in anhydrous THF (70 mL), n-BuLi (1.55 M in n-hexane, 20 mL, 32 mmol) was added dropwise over 30 min at -78°C. After stirring at -78°C for 2 hours, trimethyl borate (7.5 mL, 66.485 mmol) was added thereto, and the reaction mixture was gradually warmed up to room temperature overnight. The reaction was terminated by adding a saturated NH₄Cl solution (100 mL). After stirring at room temperature for 30 min, the reaction mixture was extracted three times with AcOEt (100 mL × 3), washed with brine, and dried over Na₂SO₄. After the solvent was concentrated under reduced pressure, the residue was purified by column chromatography (hexane : AcOEt = 5 : 1 to 2 : 1), and Compound 2 (2.9005 g, 46%, white solid) was obtained.

¹H NMR(400MHz, DMSO-d₆)δ:7.918(2H, s), 7.651-7.646(2H, d, J=2.0Hz), 7.419-7.409(1H, t, J=2.0Hz), 1.283 (18H, s).

¹³C NMR(100MHz, DMSO-d₆)δ:148.79, 128.08, 123.48, 34.43, 31.37.

HRMS (ESI-TOF, [M-H]⁻): C₁₄H₂₂BO₂, calculated value: 233.1718, measured value: 233.1731.

To a mixture of Compound 3 (103.0 mg, 0.208 mmol) and Compound 2 (89.8 mg, 0.383 mmol), Pd(OAc)₂ (1.8 mg, 0.008 mmol), PPh₃ (4.1 mg, 0.016 mmol), and K₂CO₃ (70.5 mg, 0.510 mmol, final concentration 2 M) was added. Toluene-H₂O-EtOH (2 : 1 : 2) was degassed and added to the mixture as a solvent, and then the mixture was refluxed at 85°C for 36 hours in an argon atmosphere. After being cooled to room temperature, the reaction mixture was extracted with AcOEt and dried over Na₂SO₄. The solvent was concentrated under reduced pressure, subsequently the residue was purified with an open column (n-hex : AcOEt = 15 : 1), and Compound 4 (105.0 mg, 93%, colorless oil) was obtained.

To a solution of Compound 4 (105.0 mg, 0.235 mmol) in MeOH-THF-H₂O (1 : 1 : 1, 1.5 mL), LiOH-H₂O (30.1 mg, 0.717 mmol) was added. The reaction mixture was stirred at room temperature for 3 hours. 2 M HCl was added until the reaction mixture reached pH = 2, subsequently the reaction mixture was extracted three times with CH₂Cl₂ (10 mL × 3), washed with brine, and dried over Na₂SO₄. The solvent was concentrated under reduced pressure, subsequently the residue was purified by column chromatography (hexane : AcOEt = 1 : 1), and Compound 5 (99.4 mg, 98%, white solid) was obtained.

¹H NMR(400MHz, CDCl₃)δ:7.55-7.51(2H, d, J=8.8Hz), 7.42-7.41(1H, t, J=1.6Hz), 7.38-7.38(2H, d, J=2.0Hz), 7.30-7.28(1H, dd, J=7.2, 1.6Hz), 7.22-7.18(1H, td, J=7.6, 1.6Hz), 7.03-6.99(2H, d, J=8.8Hz), 6.94-6.91(1H, dd, J=8.0, 0.8Hz), 3.03-3.00(2H, t, J=7.6Hz), 2.75-2.71(2H, t, J=7.6Hz), 1.38(18H, s).

¹³C NMR(100MHz, CDCl₃)δ:178.1, 156.8, 154.9, 151.1, 140.0, 137.4, 131.5, 130.7, 128.8, 128.0, 123.8, 121.5, 121.2, 119.1, 118.2, 35.0, 34.0, 31.5, 25.7.

HRMS (ESI-TOF, [M-H]⁻): C₂₉H₃₃O₃⁻ calculated value: 429.2435, measured value: 429.2458.

### (Step 2) Synthesis of Compound 7

Compound 7 was synthesized according to the following reaction formula.

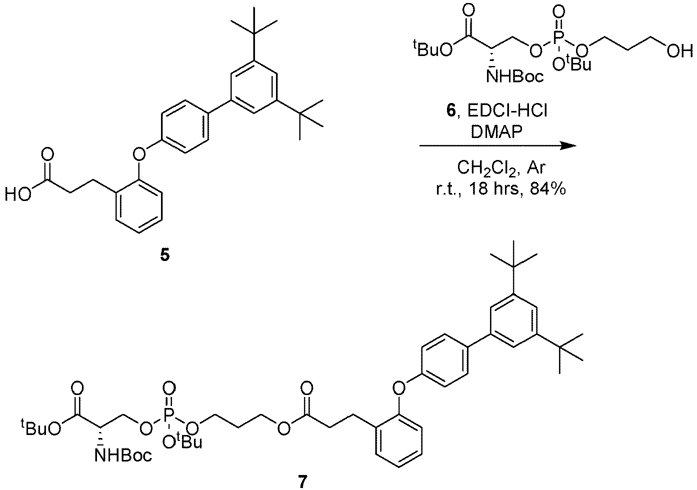

Compound 6 was synthesized according to the method described in Ikubo M., et al, J. Med. Chem. 2015, 58, 4204-4219.

To a solution of Compound 6 (100.0 mg, 0.220 mmol), Compound 5 (103.9 mg, 0.242 mmol), and DMAP (N-(4-Pyridyl)dimethylamine) (13.4 mg, 0.110 mmol) in anhydrous CH₂Cl₂ (1.0 mL), EDCI-HCl (1-Ethyl-3-(3-dimethylaminopropyl)carbodiimide Hydrochloride) ((84.2 mg, 0.439 mmol) was added. After being stirred for 18 hours at room temperature, H₂O (10 mL) was added thereto, and the mixture was extracted with CH₂Cl₂ (10 mL × 3). The solvent was concentrated under reduced pressure, subsequently the residue was purified by column chromatography (hexane : AcOEt = 2 : 1), and Compound 7 (159.6 mg, 84%, colorless oil, including diastereomers) was obtained.

¹H NMR(400MHz,CD₂Cl₂)δ:7.60-7.56(2H,d,J=8.8Hz),7.45-7.41(3H,m),7.34-7.32(1H,dd,J=7.6,1.6Hz),7.26-7.22(1H,td,J=7.6,1.6Hz),7.14-7.10(1H,td,J=7.6,1.2Hz),7.06-7.02(2H,d,J=8.8Hz),6.95-6.93(1H,dd,J=8.0,1.2Hz),5.61-5.59(1H,m),4.36-4.02(7H,m),3.03-2.99(2H,t,J=7.6Hz),2.72-2.68(2H,t,J=7.6Hz),2.01-1.95(2H,m),1.48-1.45(27H,m),1.40(18H,m).

¹³C NMR (100MHz, CD₂Cl₂)
δ:172.71,168.46,157.02,155.19,154.93,151.36,139.96,137.26,1 32.02,130.69,128.68,127.91,123.93,121.41,121.29,119.20,119. 19,83.66,83.59,82.53,79.72,67.38,64.21,64.15,60.58,54.59,34 .93,34.31,31.32,29.76,29.62,29.58,28.10,27.77,27.31,25.89.

³¹P NMR(161MHz,CD₂Cl₂)δ:-5.55,-5.69.

Anal. Calcd. for
C₄₈H₇₀NO₁₁P:C,66.42;H,8.13;N,1.61,Found:C,66.23;H,8.25;N,1.80

HRMS (ESI-TOF, [M+Na]⁺): C₄₈H₇₀NO₁₁PNa⁺ calculated value: 890.4579, measured value: 890.4589.

### (Step 3) Synthesis of Compound (II)

Compound (II) was synthesized according to the following reaction formula.

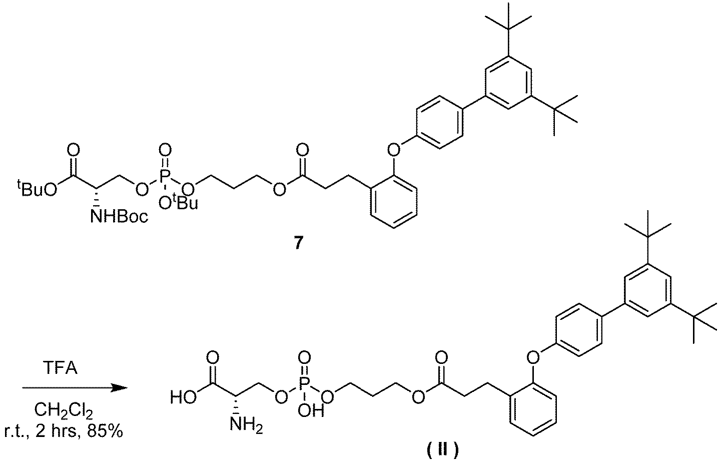

To a solution of Compound 7 (43.8 mg, 0.050 mmol) in CH₂Cl₂ (0.5 mL) at 0°C, TFA (trifluoroacetic acid) (0.5 mL) dissolved in CH₂Cl₂ (0.5 mL) was added, and the mixture was stirred at room temperature for 2 hours. After the solvent was concentrated under reduced pressure, the residue was purified by Biotage (registered trademark) Isolera^{™} reverse phase medium-pressure liquid chromatography (SHOKO, Purif-Pack (registered trademark)-EX, ODS-50 µM, size: 20; H₂O/MeCN = 90 : 10 → 40 : 60, 0.1% HCOOH), and Compound (II) (28.0 mg, 85%, white solid) was obtained.

¹H NMR(400MHz, DMSO-d₆)δ:7.64-7.60(2H, m), 7.38-7.34(4H, m), 7.27-7.23(1H, m), 7.15-7.11(1H, m), 7.02-6.99(2H, m), 6.92-6.89(1H, m), 4.06-3.96(5H, m), 3.71-3.66(2H, m), 2.89-2.85(2H, t, J=7.6Hz), 2.65-2.61(2H, t, J=7.6Hz), 1.80-1.73(2H, m), 1.33(18H, s).

³¹P NMR(161MHz, DMSO-d₆)δ:0.07.

HRMS (ESI-TOF, [M-H]⁻): C₃₅H₄₅NO₉P⁻ calculated value: 654.2837, measured value: 654.2859.

HPLC:>99%.H₂O:MeCN=40 : 60(0.1%HCOOH), t_{R}=9.880min.

### <Example 2>

### Synthesis of Compound (III)

### (Step 1) Synthesis of Compound 11

Compound 11 was synthesized according to the following reaction formula.

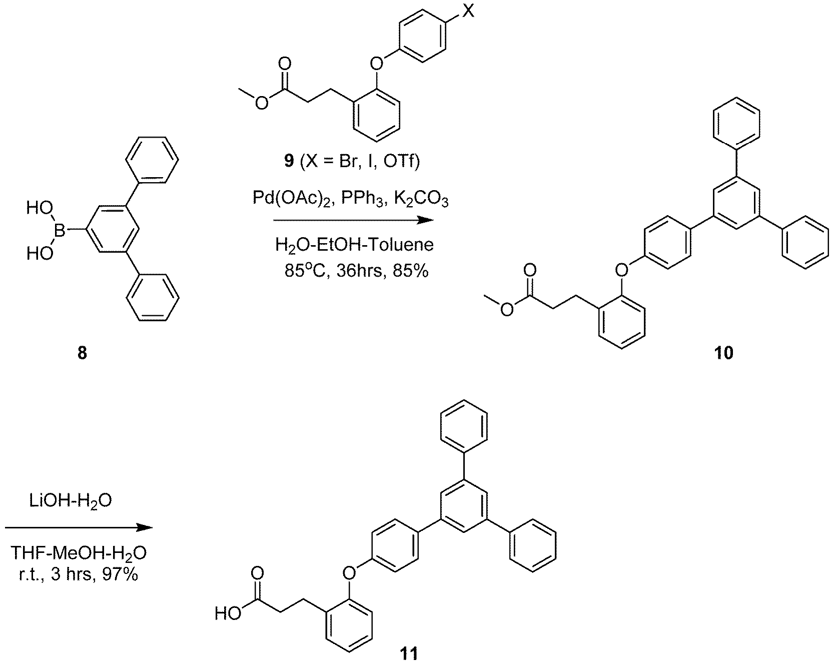

To a mixture of Compound 9 (88.5 mg, 0.219 mmol) and Compound 8 (90.1 mg, 0.32 mmol), Pd(OAc)₂ (1.5 mg, 0.007 mmol), PPh₃ (triphenylphosphine) (3.4 mg, 0.013 mmol), and K₂CO₃ (60.5 mg, 0.438 mmol, final concentration 2 M) was added. Toluene-H₂O-EtOH (2 : 1 : 2) was degassed and added to the mixture as a solvent, and then the mixture was refluxed at 85°C for 36 hours in an argon atmosphere. After being cooled to room temperature, the reaction mixture was extracted with AcOEt and dried over Na₂SO₄. The solvent was concentrated under reduced pressure, subsequently the residue was purified with an open column (n-hex : AcOEt = 15 : 1), and Compound 10 (90.2 mg, 85%, colorless oil) was obtained.

To a solution of Compound 10 (63.5 mg, 0.131 mmol) in MeOH-THF-H₂O (1 : 1 : 1, 2.1 mL), LiOH-H₂O (16.8 mg, 0.400 mmol) was added. The reaction mixture was stirred at room temperature for 3 hours. 2 M HCl was added until the reaction mixture reached pH = 2, subsequently the reaction mixture was extracted three times with CH₂Cl₂ (10 mL × 3), washed with brine, and dried over Na₂SO₄. The solvent was concentrated under reduced pressure, subsequently the residue was purified by column chromatography (hexane : AcOEt = 1 : 1), and Compound 11 (59.8 mg, 97%, white solid) was obtained.

¹H NMR(400MHz, CDCl₃)δ:7.77-7.76(3H, m), 7.71-7.69(4H, m), 7.65-7.63(2H, d, J=8.8Hz), 7.50-7.46(4H, m), 7.41-7.37(2H, m), 7.32-7.30(1H, dd, J=7.6, 1.2Hz), 7.25-7.20(1H, td, J=8.0, 1.6Hz), 7.12-7.08(1H, td, J=7.6, 1.2Hz), 7.07-7.03(2H, d, J=8.2Hz), 6.96-6.94(1H, m), 3.04-3.00(2H, t, J=7.6Hz), 2.76-2.72(2H, t, J=7.6Hz).

¹³C NMR(100MHz, CDCl₃)δ:177.9, 157.3, 154.7, 142.4, 141.7, 141.1, 135.9, 131.5, 130.7, 128.8, 128.7, 128.0, 127.6, 127.3, 124.9, 124.0, 119.3, 118.3, 33.9, 25.7.

HRMS (ESI-TOF, [M-H]⁻): C₃₃H₂₅O₃⁻ calculated value: 469.1809, measured value: 469.1813.

### (Step 2) Synthesis of Compound 12

Compound 12 was synthesized according to the following reaction formula.

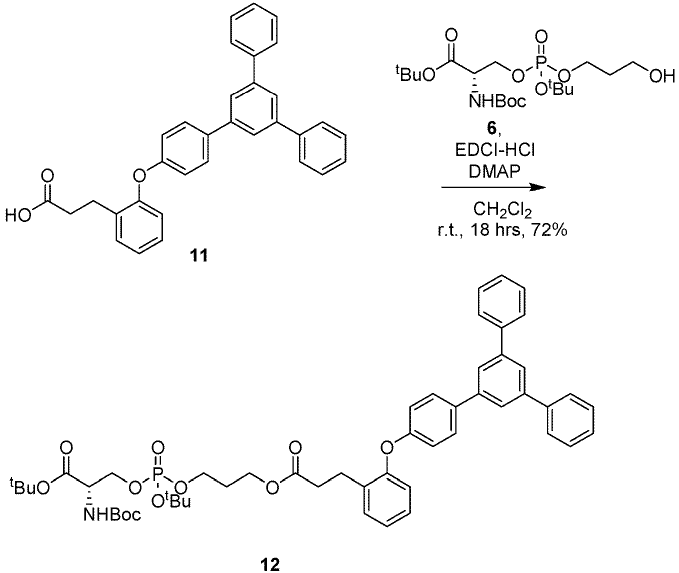

To a solution of Compound 6 (40.7 mg, 0.089 mmol), Compound 11 (43.4 mg, 0.092 mmol) and DMAP (5.7 mg, 0.047 mmol) in anhydrous CH₂Cl₂ (1.0 mL), EDCI-HCl (35.1 mg, 0.183 mmol) was added. After being stirred for 18 hours at room temperature, H₂O (10 mL) was added thereto, and the mixture was extracted with CH₂Cl₂ (10 mL × 3). The solvent was concentrated under reduced pressure, and then the residue was purified by column chromatography (hexane : AcOEt = 2 : 1), and Compound 12 (58.4 mg, 72%, colorless oil, including diastereomers) was obtained.

¹H NMR(400MHz, CD₂Cl₂)δ:7.88-7.87(3H, m), 7.82-7.76(6H, m), 7.59-7.55(4H, m), 7.50-7.45(2H, m), 7.40-7.38(1H, dd, J=7.6, 1.6Hz), 7.33-7.29(1H, td, J=7.6, 1.6Hz), 7.21-7.17(1H, td, J=7.6, 1.2Hz), 7.16-7.12(2H, d, J=8.0Hz), 7.04-7.02(1H, dd, J=7.6, 1.2Hz), 5.66-5.64(1H, m), 4.38-4.06(7H, m), 3.08-3.05(2H, t, J=7.6Hz), 2.78-2.74(2H, m), 2.06-1.99(2H, m), 1.53-1.49(27H, m).

¹³C NMR(100MHz, CD₂Cl₂)δ:172.59, 168.37, 157.50, 154.62, 142.29, 141.59, 141.01, 135.67, 132.06, 130.64, 128.83, 127.86, 127.56, 127.24, 124.72, 119.36, 118.15, 83.32, 82.42, 79.59, 67.31, 64.06, 60.49, 34.22, 31.90, 30.57, 29.66, 29.52, 29.47, 29.33, 28.00, 27.66, 25.78, 22.66.

³¹P NMR(161MHz, CD₂Cl₂) δ:-5.47, -5.62.

HRMS (ESI-TOF, [M+NH₄]⁺): C₅₂H₆₆N₂O₁₁P⁺ calculated value: 925.4399, measured value: 925.4377.

### (Step 3) Synthesis of Compound (III)

Compound (III) was synthesized according to the following reaction formula.

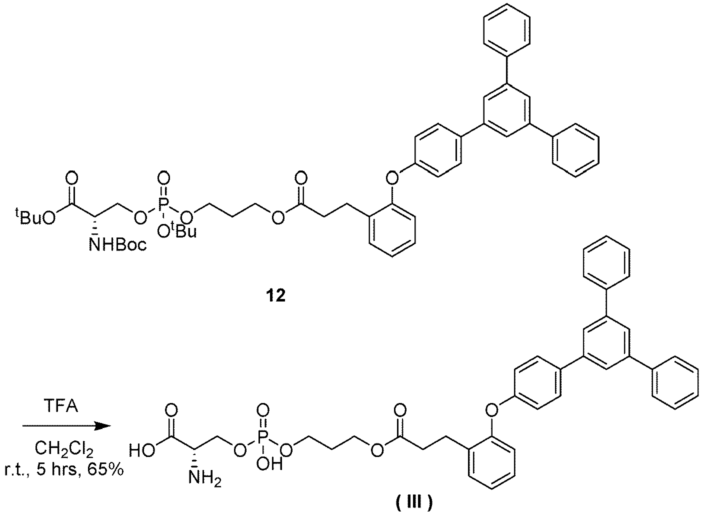

To a solution of Compound 12 (53.7 mg, 0.059 mmol) in CH₂Cl₂ (0.5 mL), TFA (0.5 mL) was added at 0°C, and the mixture was stirred at room temperature for 5 hours. After the solvent was concentrated under reduced pressure, the residue was purified by Biotage (registered trademark) Isolera^{™} reverse phase medium-pressure liquid chromatography (SHOKO, Purif-Pack (registered trademark)-EX, ODS-50 µM, size: 20; H₂O/MeCN = 80 : 20 → 40 : 60, 0.1% HCOOH), and Compound (III) (26.8 mg, 65%, white solid) was obtained.

¹H NMR(400MHz, DMSO-d6)5:7.89-7.85(9H, m), 7.53-7.49(4H, m), 7.43-7.36(3H, m), 7.30-7.26(1H, td, J=7.6, 1.6Hz), 7.17-7.13(1H, td, J=7.6, 1.2Hz), 7.06-7.03(2H, m), 6.97-6.95(1H, dd, J=8.0, 1.2Hz), 4.06-3.65(7H, m), 2.90-2.86(2H, t, J=7.6Hz), 2.66-2.62(2H, t, J=7.6Hz), 1.80-1.73(2H, quint, J=6.4Hz).

¹³C NMR: A very broad peak was detected (not published).

³¹P NMR(161MHz, DMSO-d₆)δ:0.35.

HRMS (ESI-TOF, [M-H]⁻): C₃₉H₃₇NO₁₁P⁻ calculated value: 694.2211, measured value: 694.2239.

HPLC:>98%.H₂O:MeCN=40 : 60(0.1%HCOOH), t_{R}=7.860min.

### <Example 3>

### Synthesis of Compound (XI)

### (Step 1) Synthesis of Compound 15

Compound 15 was synthesized according to the following reaction formula.

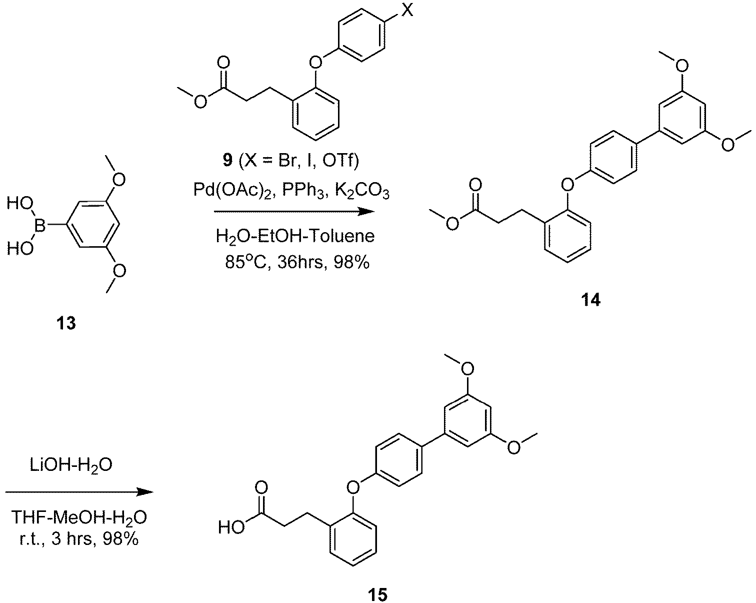

To a mixture of Compound 9 (X = OTf; 295.0 mg, 0.730 mmol) and Compound 13 (199.4 mg, 1.095 mmol), Pd(OAc)₂ (4.9 mg, 0.022 mmol), PPh₃ (11.5 mg, 0.044 mmol), and K₂CO₃ (201.8 mg, 1.460 mmol, final concentration 2 M) were added. Toluene-H₂O-EtOH (2 : 1 : 2) was degassed and added to the mixture as a solvent, and then the mixture was refluxed at 85°C for 36 hours in an argon atmosphere. After being cooled to room temperature, the reaction mixture was extracted with AcOEt and dried over Na₂SO₄. The solvent was concentrated under reduced pressure, subsequently the residue was purified with an open column (n-hex : AcOEt = 15 : 1), and Compound 14 (280.9 mg, 98%, colorless oil) was obtained.

To a solution of Compound 14 (166.5 mg, 0.425 mmol) in MeOH-THF-H₂O (1 : 1 : 1, 2.1 mL), LiOH-H₂O (53.5 mg, 1.275 mmol) was added. The reaction mixture was stirred at room temperature for 3 hours. 2 M HCl was added until the reaction mixture reached pH = 2, subsequently the reaction mixture was extracted three times with CH₂Cl₂ (10 mL × 3), washed with brine, and dried over Na₂SO₄. The solvent was concentrated under reduced pressure, subsequently the residue was purified by column chromatography (hexane : AcOEt = 1 : 1), and Compound 15 (157.7 mg, 98%, white solid) was obtained.

¹H NMR(400MHz, CDCl₃)δ:7.54-7.50(2H, d, J=8.8Hz), 7.31-7.28(1H, dd, J=7.6, 1.6Hz), 7.23-7.19(1H, td, J=7.2, 1.6Hz), 7.11-7.07(1H, td, J=7.6, 1.2Hz), 7.01-6.97(2H, d, J=8.4Hz), 6.93-6.91(1H, dd, J=8.4, 1.2Hz), 6.70-6.69(2H, d, J=2.0Hz), 6.46-6.44(1H, t, J=2.4Hz), 3.84(6H, s), 3.02-2.98(2H, t, J=7.6Hz), 2.73-2.70(2H, t, J=7.6Hz).

¹³C NMR(100MHz, CDCl₃)δ:177.9, 161.1, 157.3, 154.7, 142.8, 136.0, 131.6, 130.7, 128.5, 124.0, 119.4, 118.1, 105.3, 99.0, 55.5, 34.0, 25.7.

HRMS (ESI-TOF, [M-H]⁻): C₂₃H₂₁O₅⁻ calculated value: 377.1394, measured value: 377.1407.

### (Step 2) Synthesis of Compound 16

Compound 16 was synthesized according to the following reaction formula.

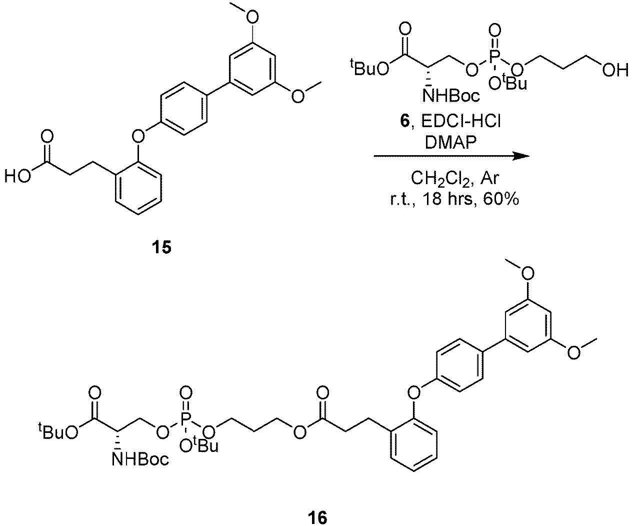

To a solution of Compound 6 (21.7 mg, 0.048 mmol), Compound 15 (21.1 mg, 0.056 mmol) and DMAP (5.7 mg, 0.047 mmol) in anhydrous CH₂Cl₂ (1.0 mL), EDCI-HCl (33.7 mg, 0.176 mmol) was added. After being stirred for 18 hours at room temperature, H₂O (10 mL) was added thereto, and the mixture was extracted with CH₂Cl₂ (10 mL × 3). The solvent was concentrated under reduced pressure, and then the residue was purified by column chromatography (hexane : AcOEt = 1 : 1), and Compound 16 (23.4 mg, 60%, colorless oil, including diastereomers) was obtained.

¹H NMR(400MHz, CD₂Cl₂)δ:7.59-7.56(2H, d, J=6.4Hz), 7.34-7.32(1H, dd, J=7.6, 1.6Hz), 7.26-7.22(1H, td, J=7.6, 1.6Hz), 7.15-7.11(1H, td, J=7.6, 1.2Hz), 7.04-7.01(2H, d, J=8.8Hz), 6.96-6.93(1H, dd, J=8.0, 1.2Hz), 6.73(2H, d, J=2.0Hz), 6.47-6.46(1H, t, J=2.4Hz), 5.61-5.59(1H, m), 4.36-4.01(7H, m), 3.86(6H, s), 3.02-2.98(2H, t, J=7.6Hz), 2.71-2.67(2H, t, J=7.6Hz), 2.00-1.94(2H, quint, J=6.4Hz), 1.48-1.45(27H, m).

¹³C NMR(100MHz, CD₂Cl₂)δ:172.66, 168.46, 161.26, 157.51, 155.17, 154.70, 142.66, 135.80, 132.12, 130.71, 128.47, 127.92, 124.08, 119.41, 118.06, 105.07, 98.93, 83.57, 82.50, 79.70, 67.33, 64.15, 64.09, 60.57, 55.43, 54.58, 34.29, 29.75, 29.61, 29.56, 28.08, 27.75, 25.84.

³¹P NMR(161MHz, CD₂Cl₂) δ:-5.49, -5.62.

HRMS (ESI-TOF, [M+NH₄]⁺): C₄₂H₆₂N₂O₁₃P⁺, calculated value: 833.3984, measured value: 833.39969.

### (Step 3) Synthesis of Compound (XI)

Compound (XI) was synthesized according to the following reaction formula.

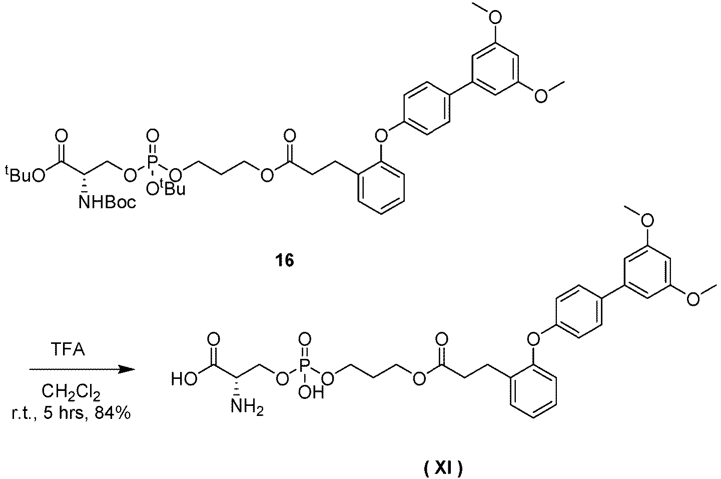

To a solution of Compound 16 (18.8 mg, 0.023 mmol) in CH₂Cl₂ (0.5 mL), TFA (0.5 mL) was added at 0°C, and the mixture was stirred at room temperature for 5 hours. After the solvent was concentrated under reduced pressure, the residue was purified by Biotage (registered trademark) Isolera^{™} reverse phase medium-pressure liquid chromatography (SHOKO, Purif-Pack (registered trademark)-EX, ODS-50 µM, size: 20; H₂O/MeCN = 80 : 20 → 40 : 60, 0.1% HCOOH), and Compound (XI) (11.7 mg, 84%, white solid) was obtained.

¹H NMR(400MHz, DMSO-d₆)δ:7.68-7.65(2H, d, J=6.8Hz), 7.37-7.28(1H, dd, J=7.6, 1.2Hz), 7.28-7.24(1H, td, J=7.6, 2.0Hz), 7.16-7.12(1H, td, J=7.2, 1.2Hz), 7.00-6.97(2H, d, J=6.4Hz), 6.93-6.91(1H, dd, J=8.0, 1.2Hz), 6.76-6.74(2H, d, J=2.0Hz), 6.47-6.46(1H, t, J=2.4Hz), 4.05-3.97(5H, m), 3.80(6H, s), 3.69-3.66(2H, m), 2.88-2.84(2H, t, J=7.6Hz), 2.64-2.60(2H, t, J=7.6Hz), 1.79-1.72(2H, quint, J=6.4Hz).

¹³C NMR: A very broad peak was detected (not published).

³¹P NMR(161MHz, DMSO-d₆)δ:0.40.

HRMS (ESI-TOF, [M-H]⁻): C₂₉H₃₃NO₁₁P⁻ calculated value: 602.1797, measured value: 602.1812.

HPLC:>96%.H₂O:MeCN=50 : 50(0.1%HCOOH), t_{R}=4.463min.

### <Example 4>

### Synthesis of Compound (XII)

### (Step 1) Synthesis of Compound 19

Compound 19 was synthesized according to the following reaction formula.

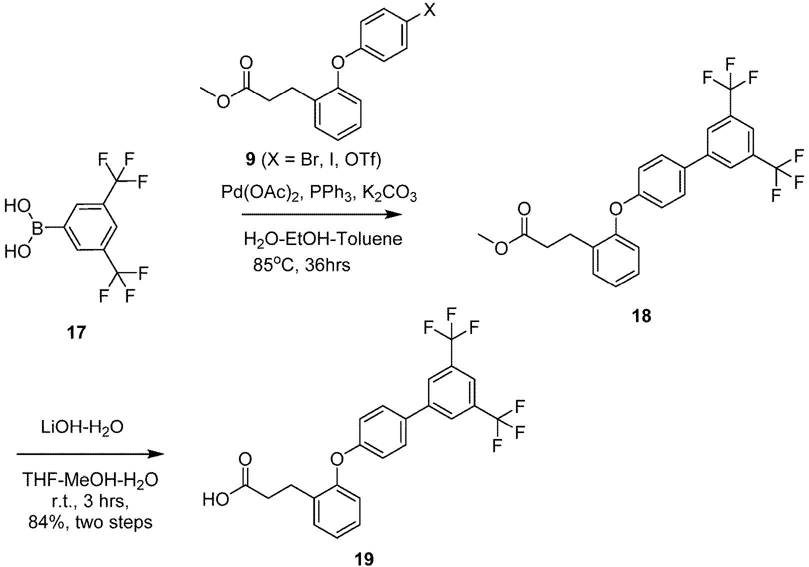

To a mixture of Compound 9 (X = Br; 100.0 mg, 0.291 mmol) and Compound 17 (112.5 mg, 0.436 mmol), Pd(OAc)₂ (2.0 mg, 0.009 mmol), PPh₃ (4.6 mg, 0.017 mmol), and K₂CO₃ (80.3 mg, 0.581 mmol, final concentration 2 M) were added. Toluene-H₂O-EtOH (2 : 1 : 2) was degassed and added to the mixture as a solvent, and then the mixture was refluxed at 85°C for 36 hours in an argon atmosphere. After being cooled to room temperature, the reaction mixture was extracted with AcOEt and dried over Na₂SO₄. The solvent was concentrated under reduced pressure, subsequently the residue was purified with an open column (n-hex : AcOEt = 15 : 1), and Compound 18 was obtained.

To a solution of Compound 18 in MeOH-THF-H₂O(1 : 1 : 1, 2.1 mL), LiOH-H₂O (36.6 mg, 0.873 mmol) was added. The reaction mixture was stirred at room temperature for 3 hours. 2 M HCl was added until the reaction mixture reached pH = 2, subsequently the reaction mixture was extracted three times with CH₂Cl₂ (10 mL × 3), washed with brine, and dried over Na₂SO₄. The solvent was concentrated under reduced pressure, subsequently the residue was purified by column chromatography (hexane : AcOEt = 2 : 1), and Compound 19 (111.0 mg, step 2 84%, white solid) was obtained.

¹H NMR(400MHz, CDCl₃)δ:7.98 (2H, m), 7.83(1H, m), 7.57-7.54(2H, d, J=8.8Hz), 7.33-7.31(1H, dd, J=7.6, 1.6Hz), 7.27-7.22(1H, td, J=8.0, 1.6Hz), 7.16-7.12(1H, td, J=7.2, 0.8Hz), 7.08-7.04(2H, d, J=8.8Hz), 6.96-6.94(1H, d, J=8.0Hz), 3.01-2.97(2H, t, J=7.6Hz), 2.73-2.70(2H, t, J=7.6Hz).

¹³C NMR(100MHz, CDCl₃)δ:179.1, 158.6, 154.2, 142.7, 132.8, 132.7(q, J²_{C-F}=33Hz), 130.8, 128.8, 128.2, 126.9(q, J⁴_{C-F}=2Hz), 124.8(q, J¹_{C-F}=272Hz), 124.6, 120.7(q, J³_{C-F}=4Hz), 119.8, 119.4, 118.3, 34.2, 25.6.

Anal. Calcd. for C₂₃H₁₆O₃F₆·0.1H₂O:C, 60.56;H, 3.58;N, 0.00, Found:C, 60.33;H, 3.98;N, 0.00.

HRMS (ESI-TOF, [M-H]⁻): C₂₃H₁₅F₆O₃⁻ calculated value: 453.0931, measured value: 453.0961.

### (Step 2) Synthesis of Compound 20

Compound 20 was synthesized according to the following reaction formula.

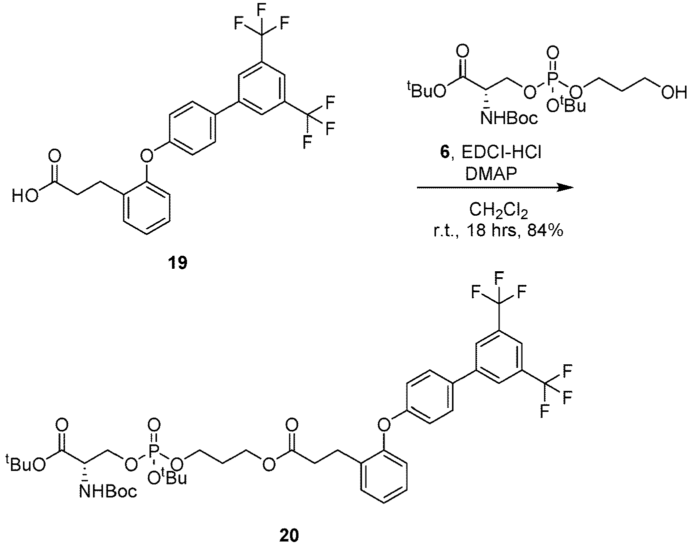

To a solution of Compound 6 (51.0 mg, 0.112 mmol), Compound 19 (55.6 mg, 0.122 mmol) and DMAP (6.8 mg, 0.056 mmol) in anhydrous CH₂Cl₂ (1.0 mL), EDCI-HCl (43.0 mg, 0.224 mmol) was added. After being stirred for 18 hours at room temperature, H₂O (10 mL) was added thereto, and the mixture was extracted with CH₂Cl₂ (10 mL × 3). The solvent was concentrated under reduced pressure, and then the residue was purified by column chromatography (hexane : AcOEt = 1 : 1), and Compound 20 (83.5 mg, 84%, colorless oil, including diastereomers) was obtained.

¹H NMR(400MHz, CD₂Cl₂)δ:8.07(2H, m), 7.89(1H, m), 7.66-7.63(2H, d, J=8.8Hz), 7.38-7.35(1H, dd, J=7.6, 2.0Hz), 7.30-7.26(1H, td, J=8.0, 1.6Hz), 7.20-7.16(1H, td, J=7.2, 1.2Hz), 7.13-7.09(2H, d, J=8.8Hz), 7.00-6.98(1H, dd, J=8.0, 1.2Hz), 5.62-5.58(1H, m), 4.36-4.02(7H, m), 3.02-2.98(2H, t, J=7.6Hz), 2.72-2.67(2H, m), 2.02-1.95(2H, m), 1.50-1.46(27H, m).

¹³C NMR(100MHz, CD₂Cl₂)δ:172.51, 168.38, 158.66, 155.09, 154.09, 142.68, 132.56, 132.34(q, J²_{C-F}=33Hz), 132.31(q, J²_{C-F}=33Hz) , 130.75, 128.69, 127.97, 126.95(q, J⁴_{C-F}=3Hz), 124.87 (q, J¹_{C-F}=271Hz), 124.49, 120.55 (q, J³_{C-F}=4Hz), 119.78, 118.16, 83.49, 83.42, 82.43, 79.61, 67.32, 67.27, 64.03, 63.99, 60.52, 54.57, 54.51, 34.19, 29.52, 29.48, 28.00, 27.66, 25.69.

³¹P NMR(161MHz, CD₂Cl₂)δ:-5.47, -5.59.

HRMS (ESI-TOF, [M+NH₄]⁺): C₄₂H₅₆F₆N₂O₁₁P⁺, calculated value: 909.3520, measured value: 909.3528.

### (Step 3) Synthesis of Compound (XII)

Compound (XII) was synthesized according to the following reaction formula.

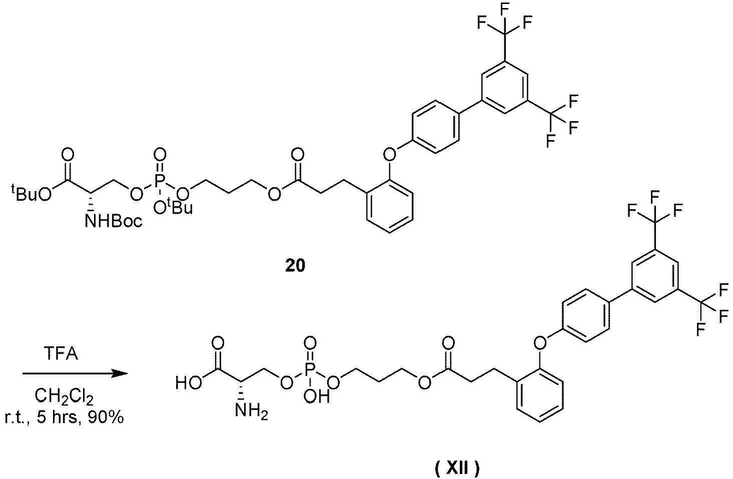

To a solution of Compound 20 (65.2 mg, 0.073 mmol) in CH₂Cl₂ (0.5 mL), TFA (0.5 mL) was added at 0°C, and the mixture was stirred at room temperature for 5 hours. After the solvent was concentrated under reduced pressure, the residue was purified by Biotage (registered trademark) Isolera^{™}reverse phase medium-pressure liquid chromatography (SHOKO, Purif-Pack (registered trademark)-EX, SI-50 µM, size: 20; CHCl₃/MeOH = 95 : 5 → 60 : 40, 0.1% HCOOH), and Compound (XII) (44.6 mg, 90%, white solid) was obtained.

¹H NMR(400MHz, DMSO-d₆)δ:8.81(2H, brs), 8.30(2H, m), 8.05(1H, m), 7.90-7.88(2H, d, J=8.8Hz), 7.39-7.37(1H, d, J=7.6Hz), 7.31-7.27(1H, td, J=8.0, 1.6Hz), 7.19-7.16(1H, t, J=7.2Hz), 7.06-7.04(2H, d, J=8.8Hz), 6.97-6.96(1H, d, J=7.6Hz), 4.06-3.66(7H, m), 2.87-2.83(2H, t, J=7.6Hz), 2.64-2.61(2H, t, J=7.6Hz), 1.79-1.73(2H, quint, J=6.4Hz).

¹³C A very broad peak was detected (not published).

³¹P NMR(161MHz, DMSO-d₆)δ:0.07.

HRMS (ESI-TOF, [M-H]⁻): C₂₉H₂₇F₆NO₉P⁻ calculated value: 678.1333, measured value: 678.1349.

### <Example 5>

### 1. Cell Culture

LX-2 cells (human hepatic stellate cells) (sigma-aldrich, reference document: Gut 2005, 54 (1), 142-151) were seeded in 12-well tissue culture plates (5×10⁴ cells/well) and cultured in Dulbecco's Modified Eagle's Medium (high glucose) (Thermo Fisher Scientific) together with 10% FBS (Fetal Bovine Serum) and 100× penicillin-streptomycin-glutamine (PSG) in a 5% CO₂ humidified incubator at 37°C. After 48 hours, the cells were stimulated with dimethyl sulfoxide (DMSO), Compound (II), and Compound (III) (5 µM) (final concentration of DMSO was 0.02%) and co-cultured for another 48 hours. Thereafter, the cells were lysed using a lysis buffer (MACHEREY-NAGEL GmbH & Co. KG).

### 2. Quantitative Real Time PCR Analysis

Total RNA of LX-2 cells was extracted using NucleoSpin RNA Plus kit (Takara Bio) according to the product protocol. Purified RNA was reverse transcribed using High-Capacity cDNA RT Kits (Thermo Fisher Scientific) according to the product protocol. PCR was performed using TB Green Premix Ex Tag II (Takara Bio) and monitored with a LightCycler96 (Roche Life Science). The number of transcripts was standardized by the number of GAPDHs, which are housekeeping genes. The primers are shown in Table 1.

**[Table 1]**

| Gene | | Forward Primer | Reverse Primer |
|---|---|---|---|
| Human | GAPDH | AAGGTGAAGGTCGGAG (SEQ ID NO:1) | AATGAAGGGGTCATTG (SEQ ID NO:2) |
| | ACTA2 | CAGCCAAGCACTGTCAGG (SEQ ID NO:3) | CCAGAGCCATTGTCACACAC (SEQ ID NO:4) |
| | COL1A1 | AAGAGGAAGGCCAAGTCGAG (SEQ ID NO:5) | CACACGTCTCGGTCATGGTA (SEQ ID NO:6) |
| | COL3A1 | AGGGGAGCTGGCTACTTCTC (SEQ ID NO:7) | AGGACTGACCAAGATGGGAA (SEQ ID NO:8) |

### 3. Results

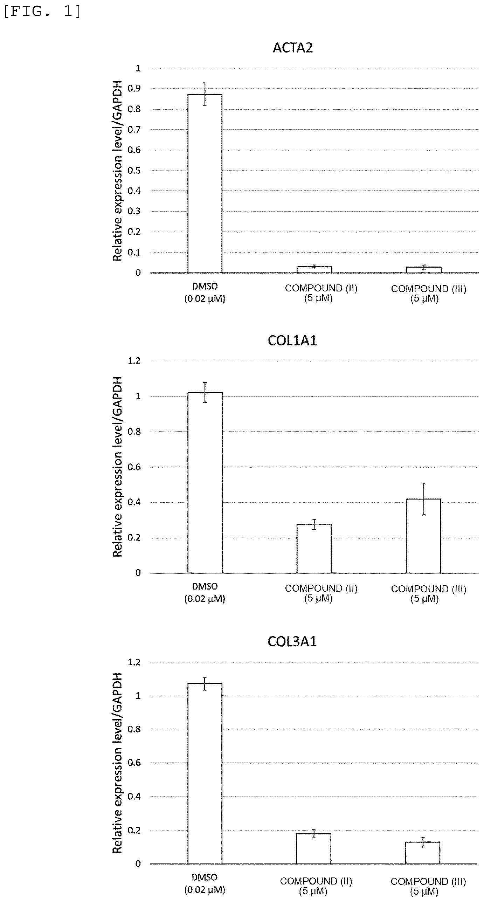

The results of allowing Compound (II) and Compound (III) to act on LX-2 cells are shown in Fig. 1. It was found that the expression levels of the marker genes for fibrosing (ACTA2, COL1A1, COL3A1) decreased.

### <Example 6>

### 1. Immunostaining

LX-2 cells were cultured in the same manner as in Example 1, except that the concentration of Compound (II) was changed to 3 µM.

LX-2 cells were fixed in Mildform (registered trademark) 10N (FUJIFILM Wako Pure Chemical Corporation) at room temperature for 10 minutes and washed with PBS. Membranes were permeabilized using 0.2% Triton X-100. The cells were blocked at room temperature for one hour using 5% skim milk or 0.3% BSA/PBS. After washing with PBS, αSMA (Dako), CollagenI (Abeam), and Hoechst33342 (Sigma-Aldrich) were each added as a first antibody, and the mixture was reacted overnight at 4°C. On the next day, the cells were washed with PBS and then reacted with Invitrogen Alexa Fluor (theromo Fisher) as a second antibody. The cells were observed under a fluorescence microscope (Keyence BZ-X810).

### 2. Results

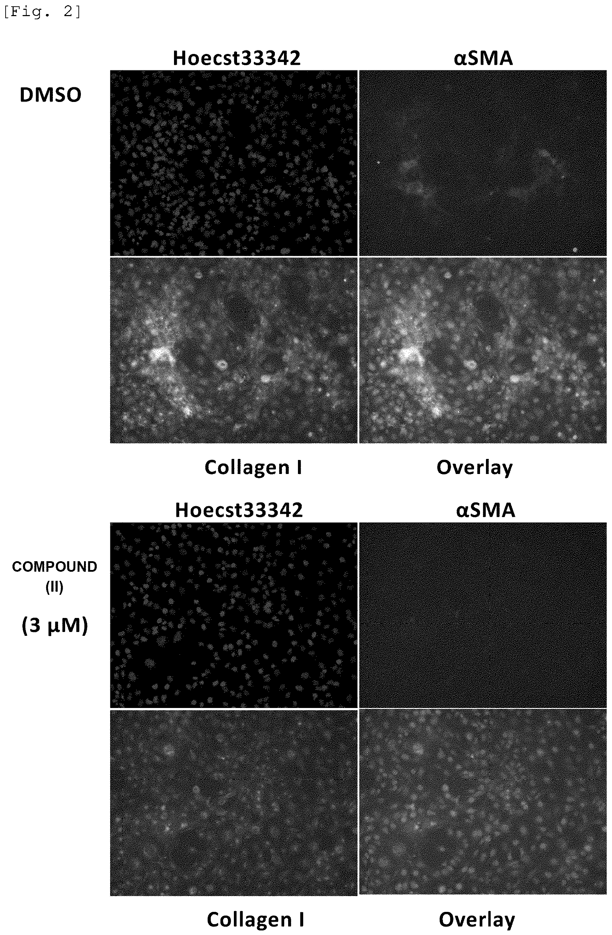

The results of immunostaining are shown in Fig. 2. αSMA (ACTA2) is shown in red, CollagenI (COL1A1) in green, and the nucleus in blue.

In the group to which DMSO was added, the cells were extensively stained in red and green, and αSMA and CollagenI were expressed. On the other hand, in the group to which Compound (II) was added, the number of regions stained in red and green was smaller than that in the group to which DMSO was added. Most of the regions were stained in blue, and the expression of αSMA and CollagenI was suppressed to an extent sufficient to suppress fibrosing.

<Example 7>

### 1. Development of Quiescent Hepatic Stellate Cell-like Cells Derived from Human iPS Cells

Quiescent hepatic stellate cell-like cells (qHSC-like cells) were induced by the methods described in Koui et al., Stem Cell Reports 2021, PCT/JP2020/006147, and PCT/JP2016/58411. qHSC-like cells derived from ACTA2-RFP receptor iPS cells were seeded in a collagen-coated 12-well plate at a density of 7×104 cells/well in Stempro-34 SFM medium (Gibco medium, theromo Fisher) supplemented with Y27632 (10 µM) and 100×PSG. After 24 hours of culture, Compound (II) was dissolved in Stempro-34 SFM medium (100×PSG), and then the solution was added such that the final concentration of Compound (II) was 1 or 2 µM. After 4 hours of incubation, the cells were lysed, and total RNA was extracted using NucleoSpin RNA Plus kit (Takara Bio).

### 2. Quantitative Real Time PCR Analysis

Total RNA derived from iPS cell-derived qHSC was extracted using NucleoSpin RNA Plus kit (Takara Bio) according to the product protocol. Purified RNA was reverse transcribed using High-Capacity cDNA RT Kits (Thermo Fisher Scientific) according to the product protocol. PCR was performed using TB Green Premix Ex Taq II (Takara Bio) and monitored with a LightCycler96 (Roche Life Science). The number of transcripts was standardized by the number of GAPDHs, which are housekeeping genes. The primers are shown in Table 2.

**[Table 2]**

| Gene | | Forward Primer | Reverse Primer |
|---|---|---|---|
| Human | GAPDH | AAGGTGAAGGTCGGAG (SEQ ID NO:9) | AATGAAGGGGTCATTG (SEQ ID NO:10) |
| | ACTA2 | CAGCCAAGCACTGTCAGG (SEQ ID NO:11) | CCAGAGCCATTGTCACACAC (SEQ ID NO:12) |
| | COL1A1 | AAGAGGAAGGCCAAGTCGAG (SEQ ID NO:13) | CACACGTCTCGGTCATGGTA (SEQ ID NO:14) |
| | COL3A1 | AGGGGAGCTGGCTACTTCTC (SEQ ID NO:15) | AGGACTGACCAAGATGGGAA (SEQ ID NO:16) |

### 3. Results

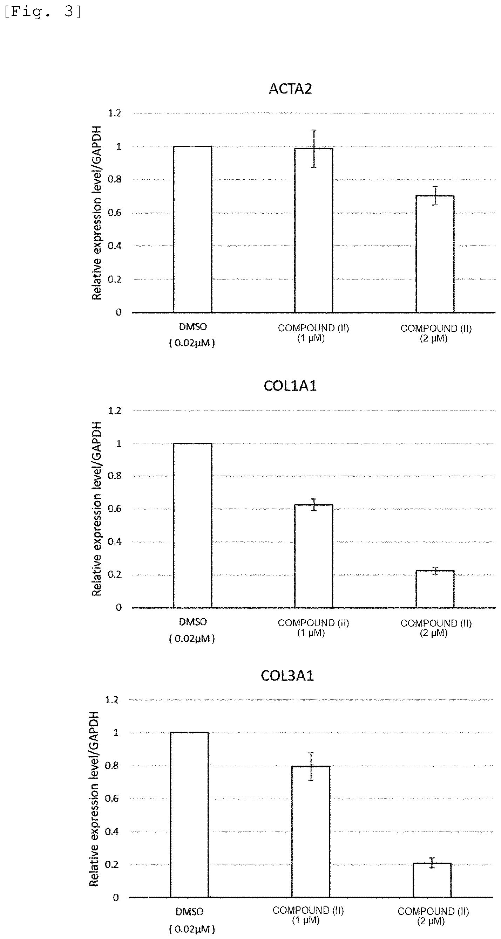

The results of allowing Compound (II) to act on qHSC cells are shown in Fig. 3. It was found that the expression levels of the marker genes for fibrosing (ACTA2, COL1A1, COL3A1) decreased.

### <Example 8>

### 1. Animal Experiments

Seven-week-old C57BL/6J mice were purchased from CLEA Japan, Inc. (Tokyo, Japan). All animals were reared in a standard specific pathogen-free room in the animal facility of the Institute for Quantitative Biosciences, University of Tokyo. For a mouse model of liver fibrosis, thioacetamide (TAA) was administered together with drinking water (0.06% v/v), starting 7 weeks before the administration of the compound. After 7 weeks of rearing with TAA-containing water, a vehicle (200 µL of PBS, containing 5% DMSO) or 20 mg/kg (200 µL of PBS, containing 5% DMSO) of Compound (II) was subcutaneously administered every 24 hours for a total period of 14 days. The mice were sacrificed 24 hours after the last injection.

### 2. Immunohistochemistry

Liver tissue samples were fixed overnight at 4°C with Zamboni's fixative (FUJIFILM Wako Pure Chemical Corporation) or Mildform (registered trademark) 10N (FUJIFILM Wako Pure Chemical Corporation). For immunofluorescence staining, the liver tissue fixed with Zamboni's fixative was washed with PBS, and the medium was replaced with a 30% (w/v) sucrose solution. Frozen tissue was prepared by embedding the tissue in a Tissue-Tek (registered trademark) O.C.T. compound (Sakura Finetek Japan) and freezing the resultant in liquid nitrogen. Liver cryosections (8 um) were mounted on slide glasses, rehydrated, and autoclaved in Target Retrieval Solution (1×, Dako) at 90°C for 20 min for epitope retrieval. The sections were blocked with 0.3% BSA/PBS for 1 hour at room temperature. After washing with PBS, αSMA (Dako), CollagenI (Bio-Rad Laboratories, Inc.), Ki67 (Santa Cruz), and Hoechst33342 (Sigma-Aldrich) were each added as a first antibody, and the mixture was reacted overnight at 4°C. On the next day, the sections were washed with PBS and then reacted with Invitrogen Alexa Fluor (theromo Fisher) as a second antibody. The section specimens were hermetically sealed and observed with a fluorescence microscope (Keyence BZ-X810). The range of positive CollagenI was quantified using Ratio_{collagen I} = Area_{collagen I}/Areaₜₒₜₐₗ.

### 3. Quantitative Real Time PCR Analysis

Total RNA of mouse liver tissue was extracted using NucleoSpin RNA Plus kit (Takara Bio) according to the product protocol. Purified RNA was reverse transcribed using High-Capacity cDNA RT Kits (Thermo Fisher Scientific) according to the product protocol. PCR was performed using TB Green Premix Ex Taq II (Takara Bio) and monitored with a LightCycler96 (Roche Life Science). The number of transcripts was standardized by the number of GAPDHs, which are housekeeping genes. The primers are shown in Table 3.

**[Table 3]**

| Gene | | Forward Primer | Reverse Primer |
|---|---|---|---|
| Mouse | GAPDH | TGTGTCCGTCGTGGATCTGA (SEQ ID NO:17) | TTGCTGTTGAAGTCGCAGGAG (SEQ ID NO:18) |
| | COL1A1 | ACATGTTCAGCTTTGTGGACC (SEQ ID NO:19) | TAGGCCATTGTGTATGCAGC (SEQ ID NO:20) |
| | COL3A1 | GGAACCTGGTTTCTTCTCACC (SEQ ID NO:21) | TAGGACTGACCAAGGTGGCT (SEQ ID NO:22) |

### 5. Results

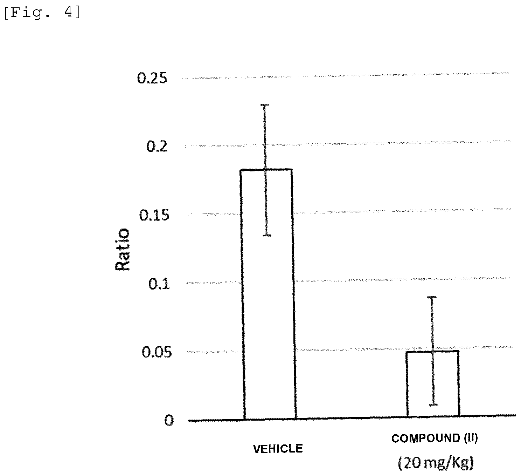

The results in the range of positive CollagenI determined by immunostaining are shown in Fig. 4. In the group on which Compound (II) was allowed to act, it was found that the positive range of CollagenI was small, and Compound (II) improved the expression of CollagenI, as compared with the vehicle.

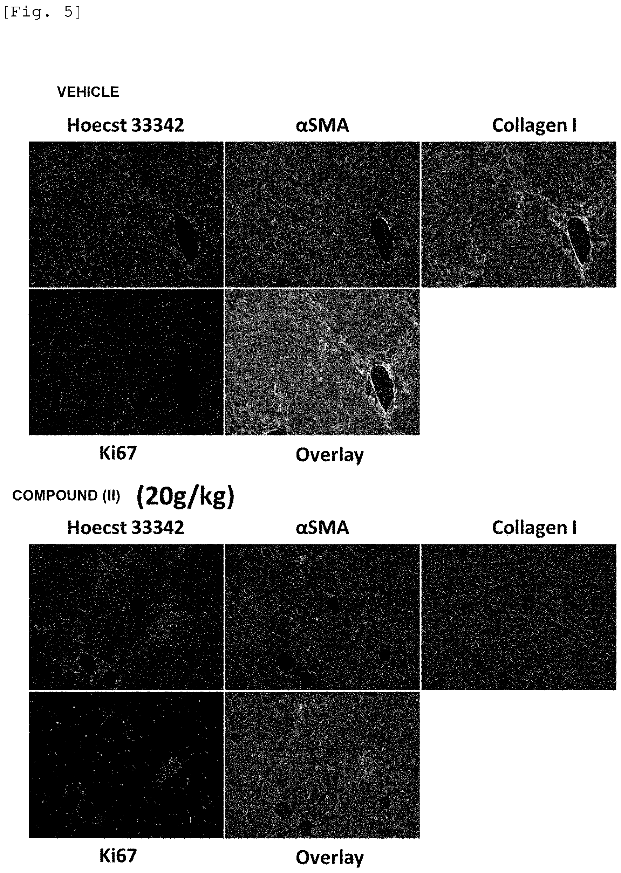

The results of immunostaining are shown in Fig. 5. The nucleus is shown in blue, αSMA (ACTA2) in green, CollagenI (COL1A1) in white, and Ki67 in red.

The group treated with the vehicle was stained extensively in green and white, and αSMA and CollagenI were expressed therein. On the other hand, in the group on which Compound (II) was allowed to act, the number of regions stained in green and white was small, and the expression of αSMA and CollagenI was suppressed to an extent sufficient to treat fibrosing, as compared with the group on which the vehicle was allowed to act.

Furthermore, it was found that the group on which Compound (II) was allowed to act had a larger area stained in red and had a normal cell proliferation capability, as compared with the group on which the vehicle was allowed to act.

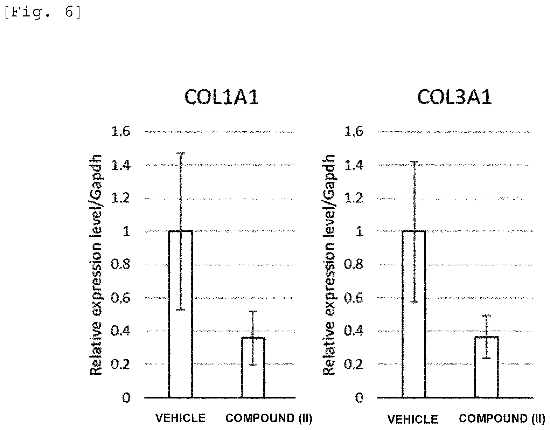

The results of allowing Compound (II) to act on a mouse with liver fibrosis are shown in Fig. 6. It was found that the expression levels of the marker genes for fibrosing (COL1A1, COL3A1) decreased.

### <Example 9>

### 1. Cell Culture

Primary cultured human renal fibroblasts (ATCC), primary cultured human lung fibroblasts (Cosmo Bio), or primary cultured human pancreatic fibroblasts (K.A.C.) were each seeded in 12-well tissue culture plates (5×10⁴ cells/well) and cultured in Minimum Essential Medium (1x) (Gibco, Thermo Fisher Scientific) together with 10% FBS (Fetal Bovine Serum) and 100× antifungal antibiotic solution (Sigma-Aldrich) in a 5% CO₂ humidified incubator at 37°C. After 48 hours, the cells were stimulated with dimethyl sulfoxide (DMSO), Compound (II), and/or Compound (III) (5 µM) (final concentration of DMSO was 0.02%) and co-cultured for another 48 hours. Cells that are activated by TGFβ1 (FUJIFILM Wako Pure Chemical Corporation), a fibroblast growth and activation factor, were stimulated with TGFβ1 (20 ng/ml) 72 hours after seeding, and after 48 hours, the cells were stimulated with dimethyl sulfoxide (DMSO), Compound (II), and/or Compound (III) (5 µM) (final concentration of DMSO was 0.02%) and co-cultured for another 48 hours. Thereafter, the cells were lysed using a lysis buffer (MACHEREY-NAGEL GmbH & Co. KG).

### 2. Quantitative Real Time PCR Analysis

Quantitative real-time PCR analysis was performed in the same manner as in Example 5.

### 3. Results

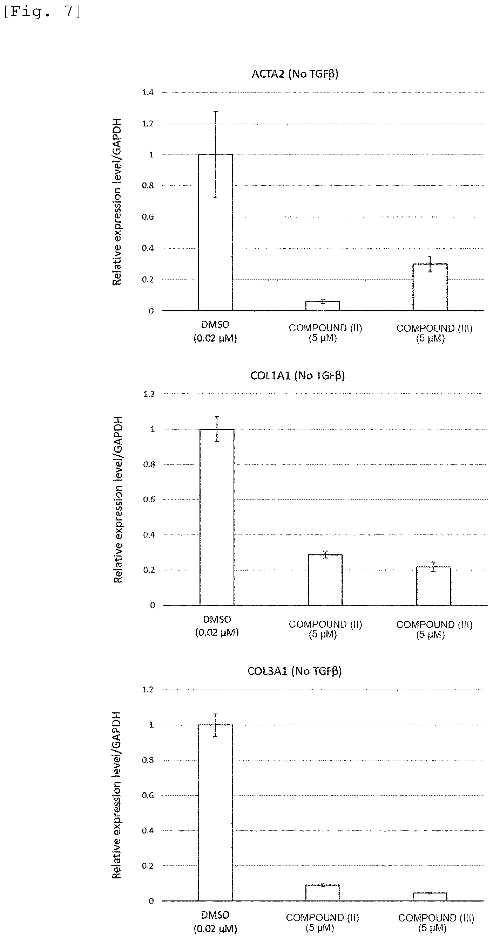

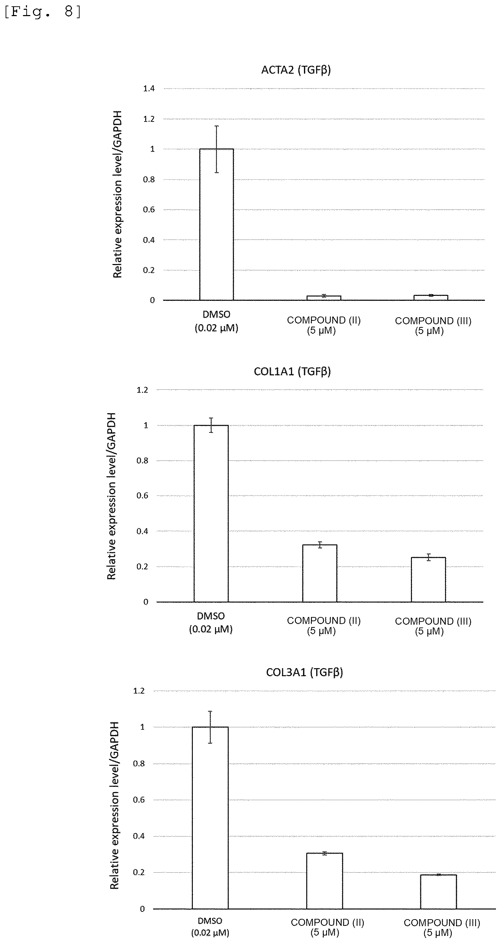

The results of allowing Compound (II) and Compound (III) to act on primary cultured human renal fibroblasts are shown in Fig. 7 and Fig. 8. Fig. 7 shows the results of the group that was not stimulated with TGFβ1, and Fig. 8 shows the results of the group that was stimulated with TGFβ1. It was found that the expression levels of the marker genes for fibrosing (ACTA2, COL1A1, COL3A1) decreased in all the groups.

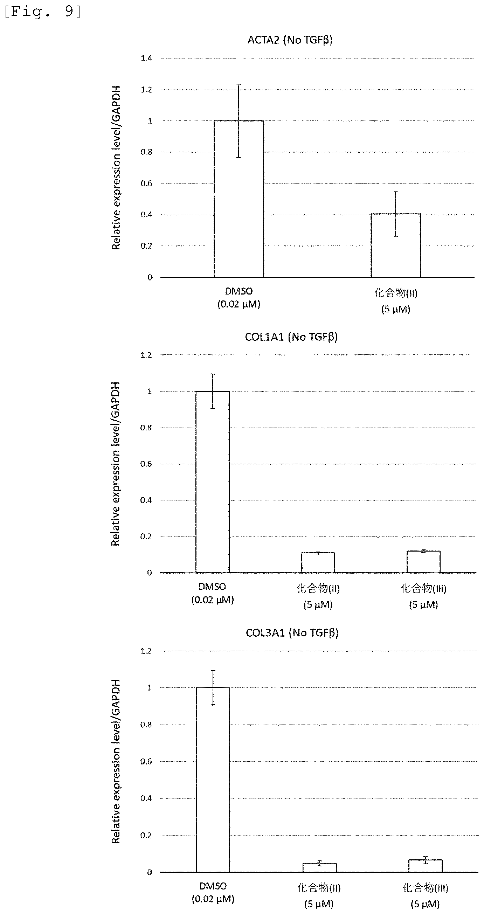

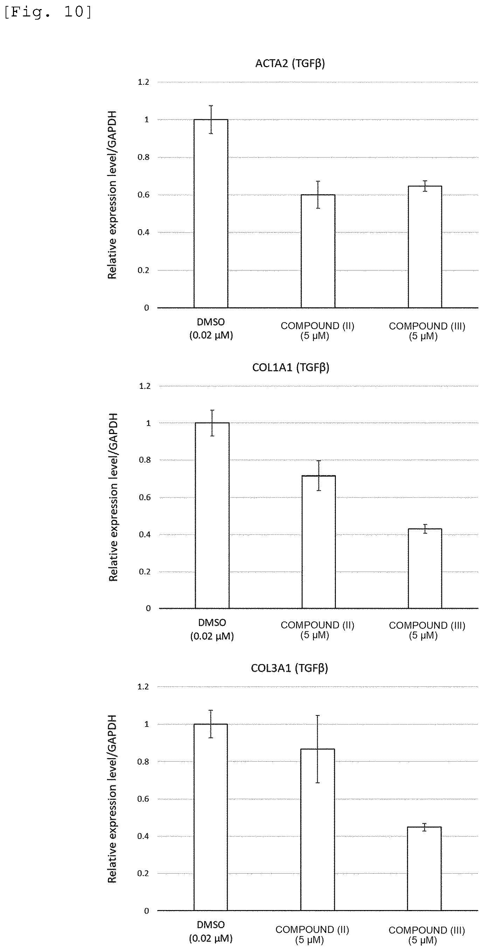

The results of allowing Compound (II) and/or Compound (III) to act on primary cultured human lung fibroblasts are shown in Fig. 9 and Fig. 10. Fig. 9 shows the results of the group that was not stimulated with TGFβ1, and Fig. 10 shows the results of the group that was stimulated with TGFβ1. It was found that the expression levels of the marker genes for fibrosing (ACTA2, COL1A1, COL3A1) decreased in all the groups.

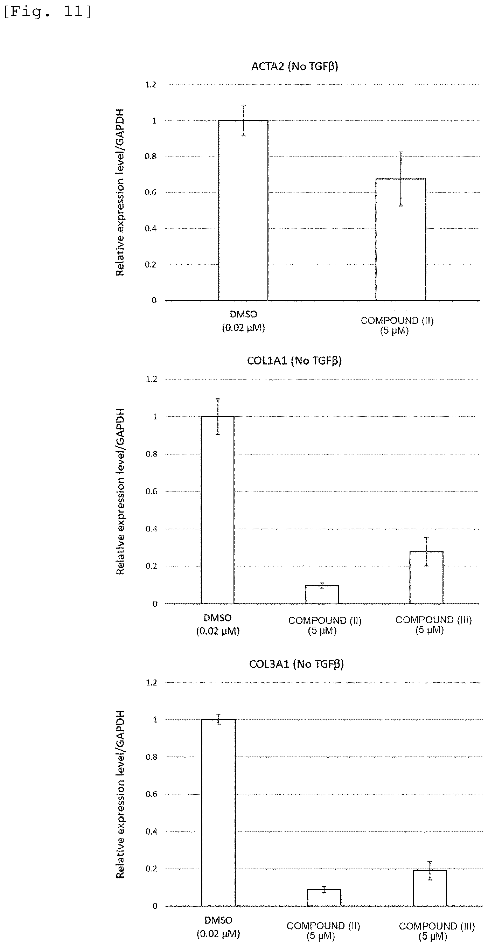

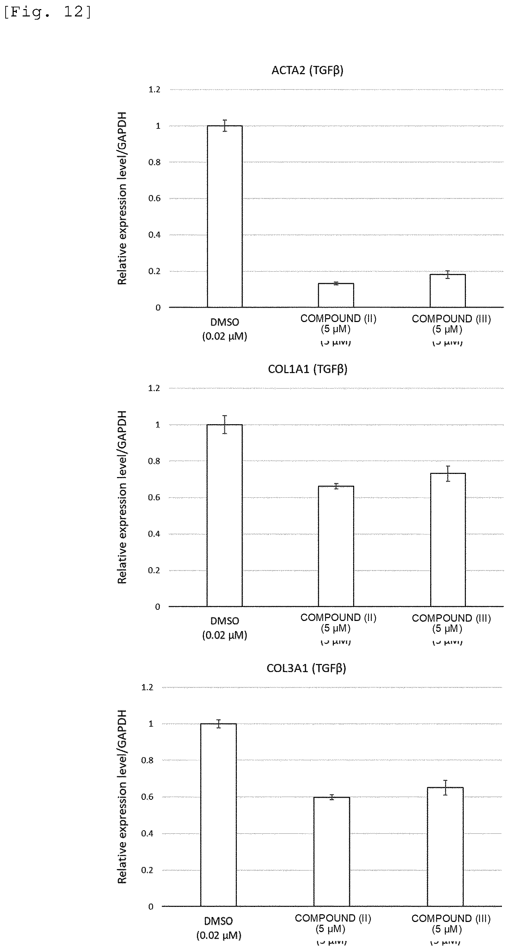

The results of allowing Compound (II) and/or Compound (III) to act on primary cultured human pancreatic fibroblasts are shown in Fig. 11 and Fig. 12. Fig. 11 shows the results of the group that was not stimulated with TGFβ1, and Fig. 12 shows the results of the group that was stimulated with TGFβ1. It was found that the expression levels of the marker genes for fibrosing (ACTA2, COL1A1, COL3A1) decreased in all the groups.

### <Example 10>

### 1. Cell Culture

Primary cultured human skin fibroblasts (Tokyo University School of Medicine) were seeded in 12-well tissue culture plates (7×10⁴ cells/well) and cultured in Dulbecco's Modified Eagle's Medium (high glucose) together with 10% FBS (Fetal Bovine Serum) and 100× penicillin-streptomycin-glutamine (PSG) in a 5% CO₂ humidified incubator at 37°C. After 48 hours, the cells were stimulated with dimethyl sulfoxide (DMSO), Compound (II), and Compound (III) (5 µM) (final concentration of DMSO was 0.02%) and co-cultured for another 48 hours. Cells activated with TGFβ1 (FUJIFILM Wako Pure Chemical Corporation) were stimulated with TGFβ1 (20 ng/ml) 72 hours after seeding, and after 48 hours, the cells were stimulated with dimethyl sulfoxide (DMSO), Compound (II), and Compound (III) (5 µM) (final concentration of DMSO was 0.02%) and co-cultured for another 48 hours. Thereafter, the cells were lysed using a lysis buffer (MACHEREY-NAGEL GmbH & Co. KG).

### 2. Quantitative Real Time PCR Analysis

Quantitative real-time PCR analysis was performed in the same manner as in Example 5.

### 3. Results

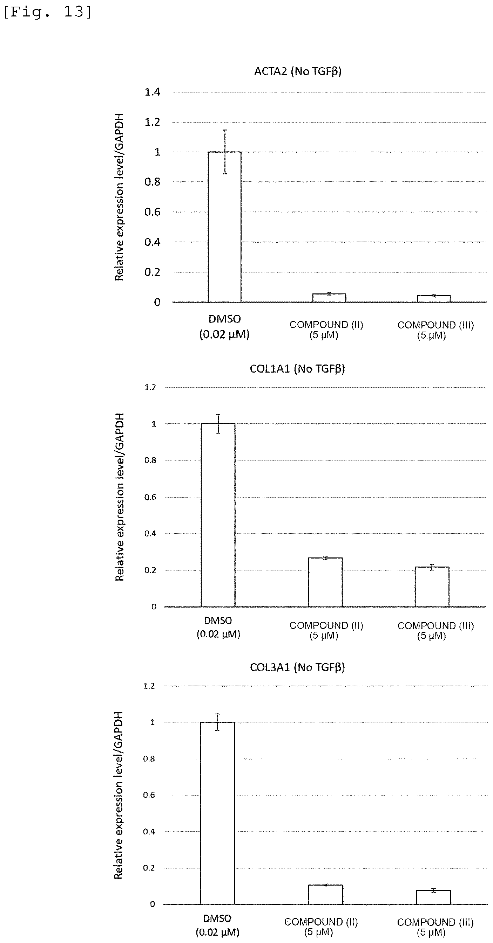

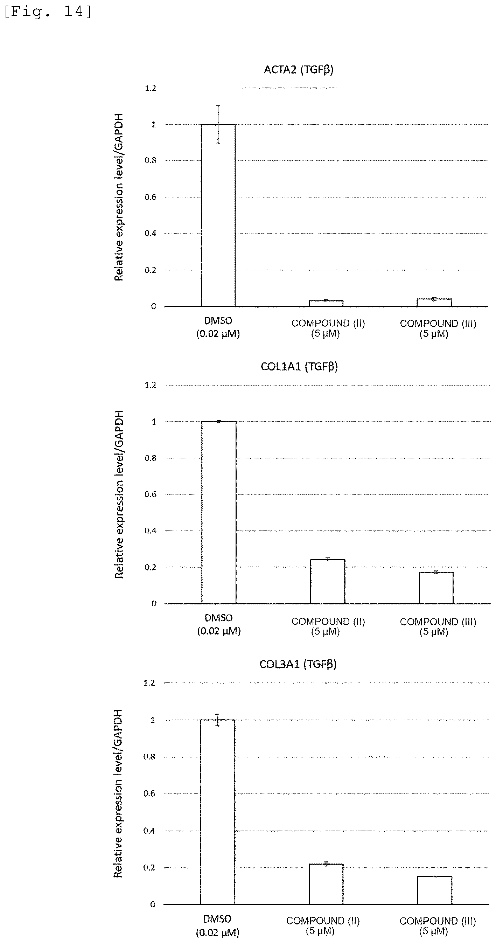

The results of allowing Compound (II) and Compound (III) to act on primary cultured human skin fibroblasts are shown in Fig. 13 and Fig. 14. Fig. 13 shows the results of the group that was not stimulated with TGFβ1, and Fig. 14 shows the results of the group that was stimulated with TGFβ1. It was found that the expression levels of the marker genes for fibrosing (ACTA2, COL1A1, COL3A1) decreased in all the groups.

## Claims

1. A compound represented by the following Formula (I) or a pharmaceutically acceptable salt thereof: 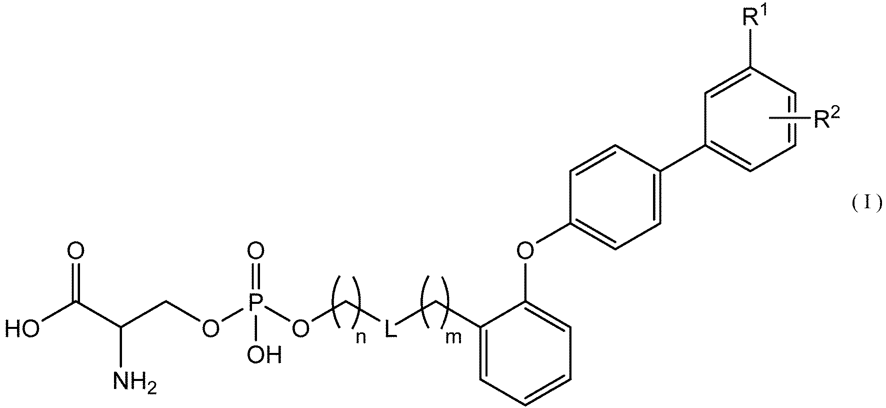 wherein in Formula (I),
L represents -O-, -NR-, -S-, -(C=O)O-, -O(C=O)-,-(C=O)NR-, -NR(C=O)-, -(C=S)NR-, -NR(C=S)-, -(C=O)S-,-S(C=O)-, -(C=S)O-, -O(C=S)-, (C=S)S-, or -S (C=S) -;
R represents a hydrogen atom or an aliphatic hydrocarbon group having 1 to 10 carbon atoms which may have a substituent;
R¹ and R² each independently represent an aliphatic hydrocarbon group having 1 to 10 carbon atoms which may have a substituent, an aromatic hydrocarbon group which may have a substituent, or -OR³;
R³ represents an aliphatic hydrocarbon group having 1 to 10 carbon atoms which may have a substituent, or an aromatic hydrocarbon group which may have a substituent; and
m and n are each independently an integer of 1 to 6.

2. The compound according to claim 1, or a pharmaceutically acceptable salt thereof, wherein R² is located at the meta-position with respect to the bonding position of R¹.

3. The compound according to claim 1 or 2, or a pharmaceutically acceptable salt thereof, wherein R¹ and R² are identical.

4. The compound according to any one of claims 1 to 3, or a pharmaceutically acceptable salt thereof, wherein R¹ and R² are each independently ethyl, isopropyl, t-butyl, phenyl, methylphenyl, benzyl, trifluoromethyl, or methoxy.

5. A compound represented by the following Formula (II) or the following Formula (III), or a pharmaceutically acceptable salt thereof.

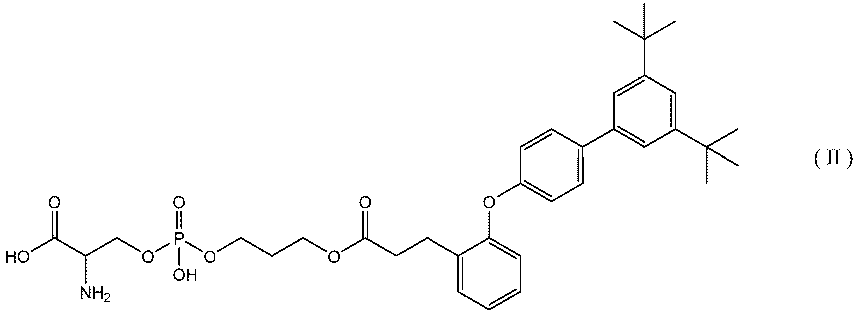

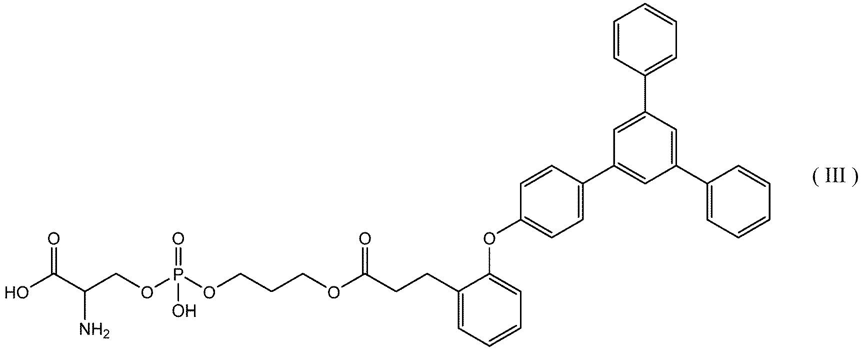

6. A pharmaceutical composition for treating or preventing fibrosis, comprising the compound according to any one of claims 1 to 5 or a pharmaceutically acceptable salt thereof.

7. A pharmaceutical composition for treating or preventing fibrosis, comprising a compound represented by the following Formula (II) or the following Formula (III), or a pharmaceutically acceptable salt thereof.

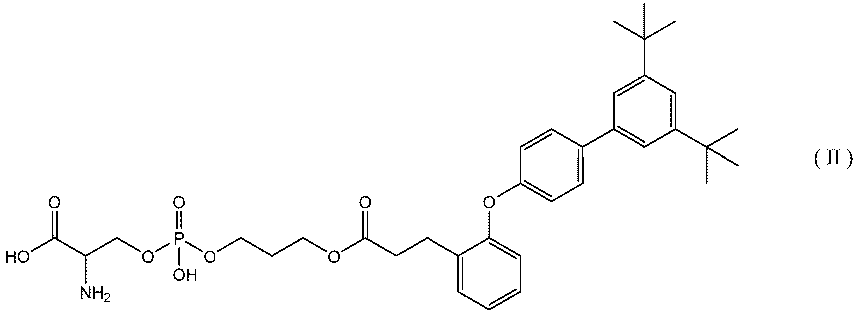

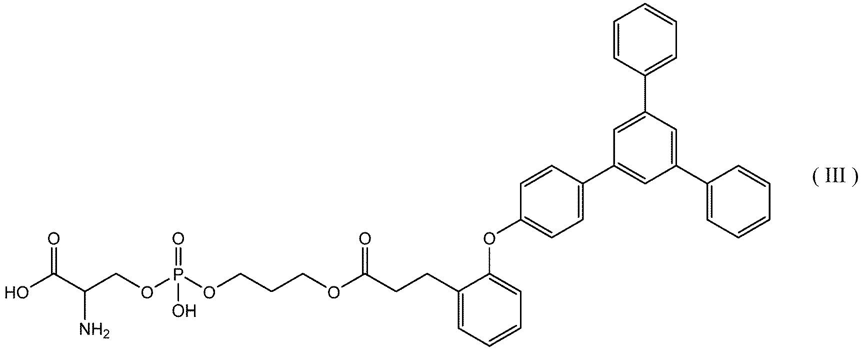

8. The pharmaceutical composition according to claim 6 or 7, wherein the fibrosis is fibrosis in a heart, an eye, a brain, a digestive organ, a skin, a lung, a kidney, a hematopoietic organ, a retroperitoneum, a mediastinum, or a joint.

9. The pharmaceutical composition according to any one of claims 6 to 8, wherein the fibrosis is cardiac fibrosis, preretinal fibrosis, gastrointestinal fibrosis, intestinal fibrosis, Crohn's disease, liver fibrosis, liver cirrhosis, chronic liver disease, scleroderma, idiopathic interstitial pneumonia, lung fibrosis, kidney fibrosis, nephrogenic systemic fibrosis, chronic kidney disease, chronic pancreatitis, cystic fibrosis, myelofibrosis, retroperitoneal fibrosis, mediastinal fibrosis, or joint fibrosis.
